# EUROPEAN PATENT SPECIFICATION

(11) **EP 1 773 230 B1**
(45) Date of publication and mention of the grant of the patent: **28.09.2016**
(21) Application number: 05770789.5
(22) Date of filing: 03.08.2005
(51) Int. Cl.: A61B 1/00, A61B 34/00

(54) **MAGNETIC GUIDING MEDICAL SYSTEM**
MAGNETISCH LEITENDES MEDIZINISCHES SYSTEM
SYSTEME MEDICAL A GUIDAGE MAGNETIQUE

(30) Priority: 03.08.2004 JP 2004227214; 30.06.2005 JP 2005192628
(43) Date of publication of application: 18.04.2007
(73) Proprietor: Olympus Corporation, Shibuya-ku Tokyo 151-0072 (JP)
(72) Inventor: KAWANO, Hironao c/o Olympus Corporation, Hachioji-shi, Tokyo 192-8507 (JP); SATO, Ryoji c/o Olympus Corporation, Hachioji-shi, Tokyo 192-8507 (JP)
(74) Representative: von Hellfeld, Axel
(86) International application number: PCT/JP2005/014608
(87) International publication number: WO 2006/014011

(56) References cited:
- WO-A-02/49705
- WO-A1-03/086190
- US-A1- 2001 021 805
- US-A1- 2001 038 683
- US-A1- 2001 047 129
- US-A1- 2002 186 520
- US-A1- 2004 019 447
- US-A1- 2004 064 153

## Description

### Technical Field

The present invention relates to a magnetic guiding medical system that magnetically guides a medical apparatus which is inserted in the living body.

### Background Art

U.S. Patent Publication No. 3358676 discloses a magnetic propulsion apparatus for guiding through the body cavity or the like, in which nine electromagnets are disposed on the plane and further electromagnets are disposed on the plane facing each other.

Further, PCT WO 02/49705 description discloses a generating apparatus of the three-dimensional magnetic field on the top of two parallel pairs of electromagnets by orthogonally laminating the two pairs of electromagnets and disposing one electromagnet to surround one of the two pairs.

In the conventional example, with the structure in which the electromagnets are disposed on the plane and the three-dimensional magnetic field is generated on the top thereof, the space for ideally generating the magnetic field is extremely limited.

Therefore, in the magnetic guiding medical system, upon guiding, by the magnetic generating device, the medical apparatus having an insertion unit into the body and a permanent magnet in the insertion unit into the body, there is such a problem that the area for guiding the insertion unit is not sufficiently ensured and the precision of the generated magnetic field at the position for generating the magnetic field deteriorates.

Document US 2004/006453 A1 concerns an efficient magnet system for magnetically-assisted surgery. In one embodiment, a tiny magnet on the end of a catheter or a guide wire is magnetically navigated into an aneurysm in a brain.

### Disclosure of Invention

The present invention concerns a magnetic guiding medical system having the features of claim 1.

According to the present invention, a magnetic guiding medical system comprises: a medical apparatus having an insertion unit that is inserted in the body cavity of the living body; a position/posture detecting unit that detects at least one of the position and the posture of the insertion unit; a magnetic field generating unit having at least three electromagnets that are axial-symmetrically arranged on the substantially plane and have the magnetizing directions in the orthogonal direction of the plane; a magnetic field control unit that controls the magnetic field generated by the magnetic field generating unit; a position/posture varying unit that changes a relative position/posture between the magnetic field generating unit and the insertion unit in accordance with information on the position and the posture of the insertion unit obtained by the position/posture detecting unit; and
a magnetic field operated unit arranged to the insertion unit;
wherein the magnetic field generated by the magnetic field generating unit operates on the magnetic field operated unit, thereby guiding the medical apparatus.

With the above-mentioned structure, the changing operation of a relative position/posture between the magnetic generating unit and the insertion unit is controlled so that the position of the insertion unit of the medical apparatus is recognized and the optimum magnetic field is generated at the recognized position, thereby setting a wide controllable region in the living body.

### Brief Description of the Drawings

Fig. 1 is a diagram showing the overall structure of a magnetic guiding medical system according to a first embodiment of the present invention;
Fig. 2 is a block diagram showing the internal structure of a planar moving mechanism and a control unit shown in Fig. 1;
Fig. 3 is a diagram showing the internal structure of a capsule medical apparatus;
Fig. 4 is a diagram showing a receiving antenna unit which receives an electromagnetic field that is sent by wireless manner from the capsule medical apparatus;
Fig. 5 is a block diagram showing the structure of a signal processing system in the control unit outside the body;
Fig. 6 is a diagram showing the detailed structure of the receiving antenna unit shown in Fig. 6;
Fig. 7 is a diagram showing an example of the structure of a receiving antenna unit according to a modification;
Fig. 8 is a diagram showing the structure of a magnetic field generating unit whose current supplied by a current control unit is controlled;
Fig. 9 is a plan view showing the specific structure of the magnetic field generating unit;
Fig. 10 is a perspective view showing the specific structure of the magnetic field generating unit;
Fig. 11 is a sectional view showing the specific structure of the magnetic field generating unit;
Fig. 12 is a characteristic diagram showing the strength of the magnetic field which is generated on the central axis by an electromagnet for generation in the axial directions, forming the magnetic field generating unit;
Fig. 13 is a characteristic diagram showing the influence caused by the deviation of the strength of the magnetic field from that to be originally generated on the axes on the deviated angle between the magnetic field direction to be actually generated and the magnetic field direction to be originally generated;
Fig. 14 is a schematic diagram showing a magnetic guidable region;
Fig. 15 is an explanatory diagram showing the state of propelling the capsule medical apparatus by applying the rotating magnetic field thereto;
Fig. 16 is an explanatory diagram showing the state of the control operation for keeping the capsule medical apparatus within a guidable region by moving the magnetic field generating unit based on positional information by the wireless electromagnetic waves from the capsule medical apparatus;
Fig. 17 is a diagram showing the state of keeping the capsule medical apparatus just on the top of the magnetic field generating unit;
Fig. 18 is a diagram showing the characteristics of the generated magnetic field per unit current relative to the distance between the magnetic poles of electromagnets 3 to 5 stored in a generated magnetic field storing unit and the magnetic field generating unit;
Fig. 19A is a side view showing the structure of a planar moving mechanism unit according to a first modification;
Fig. 19B is a front view showing the structure of the planar moving mechanism unit according to the first modification;
Fig. 20 is a perspective view showing the structure of a planar moving mechanism unit according to a second modification;
Fig. 21A is a plan view showing the structure for calculating positional information by using a plurality of ultrasonic probes;
Fig. 21B is a front view showing the structure for calculating the positional information by using a plurality of ultrasonic probes;
Fig. 21C is a diagram showing an ultrasonic image obtained by the plurality of ultrasonic probes;
Fig. 22A is a diagram showing the state of calculating the three-dimensional position by rotating the ultrasonic probes;
Fig. 22B is a diagram showing the state of calculating the three-dimensional position by using an array ultrasonic probe;
Fig. 23 is a diagram showing the structure for calculating the position by attaching the ultrasonic probe onto the magnetic field generating unit in a bed;
Fig. 24 is a diagram showing the attachment structure of the ultrasonic probe that is detachable;
Fig. 25 is a diagram showing the structure for calculating the position by an ultrasonic probe array attached to cover the body surface of a patient;
Fig. 26 is a diagram showing the structure having a material for reflecting ultrasonic waves on the capsule medical apparatus;
Fig. 27 is a diagram showing an example of the arrangement of the magnetic field generating unit in a chair;
Fig. 28 is a perspective view showing an example of the planar moving mechanism;
Fig. 29 is a diagram showing a measurement result of the generated magnetic field in the case of changing the current flowing to the electromagnet in the center;
Fig. 30 is a diagram showing the overall structure of a magnetic guiding medical system according to a second embodiment of the present invention;
Fig. 31A is a plan view showing the structure of a magnetic field generating unit;
Fig. 31B is a sectional view showing the structure of the magnetic field generating unit;
Fig. 32A is a diagram showing the structure of an endoscope on the distal-end side thereof;
Fig. 32B is a diagram showing the structure of an endoscope on the distal-end side thereof according to a modification;
Fig. 33 is an explanatory diagram of operation for guiding the distal end of the endoscope within a guidable region;
Fig. 34 is an explanatory diagram of the guiding operation within the guidable region by moving the electromagnet in the center;
Fig. 35 is a sectional view showing the magnetic field generating unit with the structure for moving the electromagnet in the center;
Fig. 36 is a diagram showing a measurement result of the generated magnetic field in the case of changing the height of the electromagnet in the center;
Fig. 37 is a diagram schematically showing the structure of a magnetic guiding medical system according to a third embodiment of the present invention;
Fig. 38A is a diagram showing a capsule medical apparatus including a magnet magnetized in the axial (longitudinal) direction;
Fig. 38B is a diagram showing a capsule medical apparatus including a magnet magnetized in the diameter direction;
Fig. 39 is a plan view showing the structure of a magnetic field generating unit;
Fig. 40 is a plan view showing the structure of a magnetic field generating unit according to a modification of the third embodiment;
Fig. 41A is a sectional view schematically showing an electromagnet forming the magnetic field generating unit;
Fig. 41B is a sectional view schematically showing the electromagnet shown in Fig. 41A wherein a ferromagnetic member is arranged to the bottom side;
Fig. 41C is a sectional view schematically showing the electromagnet shown in Fig. 41A, wherein a ferromagnetic member with the same size as the bottom size is arranged on the bottom side;
Fig. 42 is a diagram showing measurement results of the generated magnetic field with the arrangement of the ferromagnetic member and without it shown in Fig. 41A;
Fig. 43 is a sectional view schematically showing a magnetic field generating unit having the ferromagnetic member on the bottom side of the overall electromagnet;
Fig. 44 is a sectional view schematically showing a magnetic field generating unit having the ferromagnetic member on the top of the electromagnet in the center on the magnetic field generating side thereof;
Fig. 45 is a diagram showing measurement results of the generated magnetic field with the arrangement of the ferromagnetic member on the top surface of the electromagnet in the center and without it;
Fig. 46 is a sectional view schematically showing the electromagnet in the center having a caved portion in the center of the core of the electromagnet in the longitudinal direction thereof;
Fig. 47 is a sectional view schematically showing the electromagnet in the center having a large core cross-sectional area on the top of the magnetic field generating side thereof;
Fig. 48 is a sectional view schematically showing the magnetic field generating unit having the ferromagnetic member on the top surface of the electromagnet on the peripheral side on the magnetic field generating side thereof;
Fig. 49 is a diagram showing the structure of a magnetic guiding medical system according to a fourth embodiment of the present invention;
Fig. 50 is a diagram showing the structure of the facing arrangement of a pair of magnetic field generating units;
Fig. 51 is a diagram showing one of the pair of magnetic field generating units having the facing arrangement of only the electromagnet in the center shown in Fig. 50;
Fig. 52 is a diagram showing the schematic characteristics of the generated magnetic field in the case shown in Fig. 51;
Fig. 53A is a sectional view showing the magnetizing directions of the electromagnets;
Fig. 53B is a diagram schematically showing the magnetizing directions of the electromagnets;
Fig. 54A is a diagram showing the structure of a distal end of a catheter including a magnet magnetized in the axial direction;
Fig. 54B is a diagram showing the distal end of a catheter including the magnet magnetized in the diameter direction;
Fig. 55 is a diagram showing the structure of a magnetic guiding medical system according to a fifth embodiment of the present invention;
Fig. 56A is a side view showing the structure of a peripheral portion of a bed;
Fig. 56B is a front view showing the structure of the peripheral portion of the bed;
Fig. 57 is a diagram showing a capsule medical apparatus including a marker coil;
Fig. 58 is a diagram showing an example of the arrangement structure of a magnetic field generating unit, a drive coil, and the like;
Fig. 59 is an explanatory diagram showing the structure of a position/posture detecting mechanism of the capsule medical apparatus;
Fig. 60A is a diagram showing a drive coil arranged on the bed side according to one modification of the case shown in Fig. 58;
Fig. 60B is a diagram showing a sensing coil arranged on the bed side according to another modification of the case shown in Fig. 58;
Fig. 60C is a diagram showing a drive coil and a sensing coil arranged on the bed side according to another modification of the case shown in Fig. 58;
Fig. 61 is a flowchart showing the operation of a magnetic guiding method according to the embodiments;
Fig. 62 is an explanatory diagram for the control operation of the position of the bed by using calibration data;
Fig. 63 is an explanatory diagram of the operation for the feedback control of a magnetic field generating unit by a magnetic field control unit according to one modification;
Fig. 64A is a diagram showing the structure of a magnetic field generating unit according to a modification; and
Fig. 64B is a plan view showing the structure of a core portion and an auxiliary magnetic pole portion shown in Fig. 64A.

### Best Mode for Carrying Out the Invention

Hereinbelow, embodiments of the present invention will be described with reference to the drawings.

### (First embodiment)

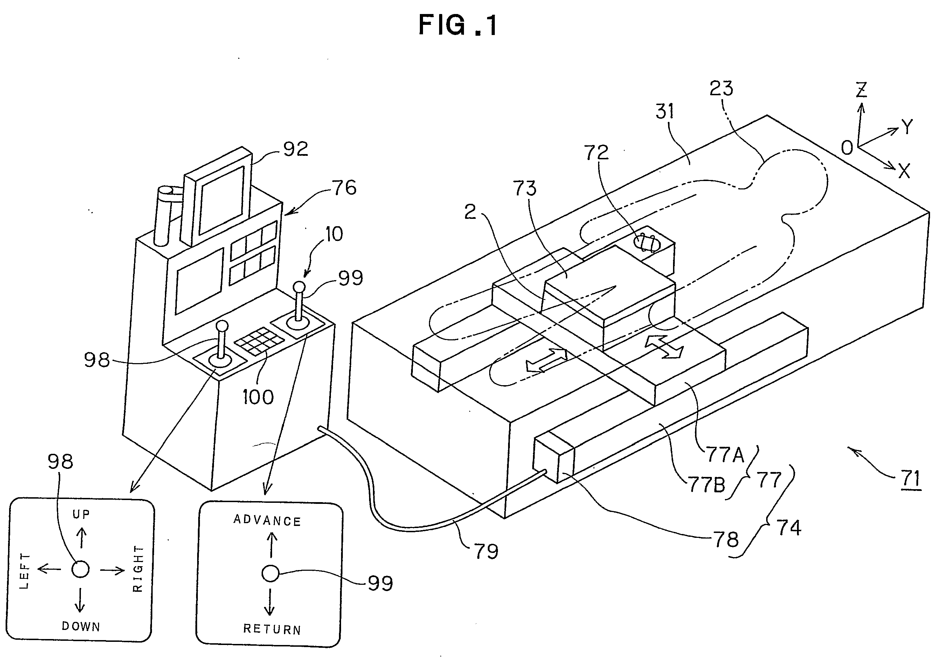

A first embodiment of the present invention will be described with reference to Figs. 1 to 29.

Referring to Fig. 1, a magnetic guiding medical system 71 comprises: a capsule medical apparatus 72 which examines, by endoscopy, a patient 23, as shown by an alternate long and two short dashes line, laid on the top of a bed 31; a magnetic field generating unit 2 which generates the magnetic field for guiding the capsule medical apparatus 72 and a receiving antenna unit 73 arranged in a casing inside the bed 31; a planar moving mechanism unit 74 (serving as position/posture varying means) which moves the receiving antenna unit 73 and the magnetic field generating unit 2 on the plane; and a control unit 76 which is arranged outside the bed 31, controls the magnetic field generated by the magnetic field generating unit 2, and has an instruction/operation unit 10, serving as a user interface.

Referring to Fig. 1, on the three-dimensional orthogonal coordinate system with the X axis, serving as the width direction on the top surface of the bed 31, the Y axis, serving as the longitudinal direction thereof, and the Z axis above in the vertical direction of the top surface of the bed 31, a planar moving mechanism 77 forming the planar moving mechanism unit 74 comprises an X axial direction moving-stage 77A and a Y axial direction moving-stage 77B. The receiving antenna unit 73 and the magnetic field generating unit 2 are placed on the X axial direction moving-stage 77A which is moved in the X axial direction, and the X axial direction moving-stage 77A is placed on the Y axial direction moving-stage 77B which is moved in the Y axial direction.

The receiving antenna unit 73 and the magnetic field generating unit 2 are held movably in the X axial direction and the Y axial direction by the planar moving mechanism 77. The receiving antenna unit 73 and the magnetic field generating unit 2 may be accommodated in a common casing.

The planar moving mechanism unit 74 comprises: the X axial direction moving-stage 77A; the Y axial direction moving-stage 77B; and an in-planar position control unit 78 which controls the positions on the plane of the X axial direction moving-stage 77A and of the Y axial direction moving-stage 77B.

As will be described later, according to the first embodiment, a magnetic field generating region controllable by the magnetic field by the single magnetic field generating unit 2 is extremely limited. However, the planar moving mechanism 77 moves the magnetic field generating unit 2, thereby widening the guidable region.

The control operation of current flowing to electromagnet units 3 to 5 forming the magnetic field generating unit 2 generates the optimum magnetic field near the capsule medical apparatus 72, which is the guiding and control target, inserted in the patient 23 and further widens the guidable region.

The in-planar position control unit 78 is connected to an external control unit 76 via a signal cable 79.

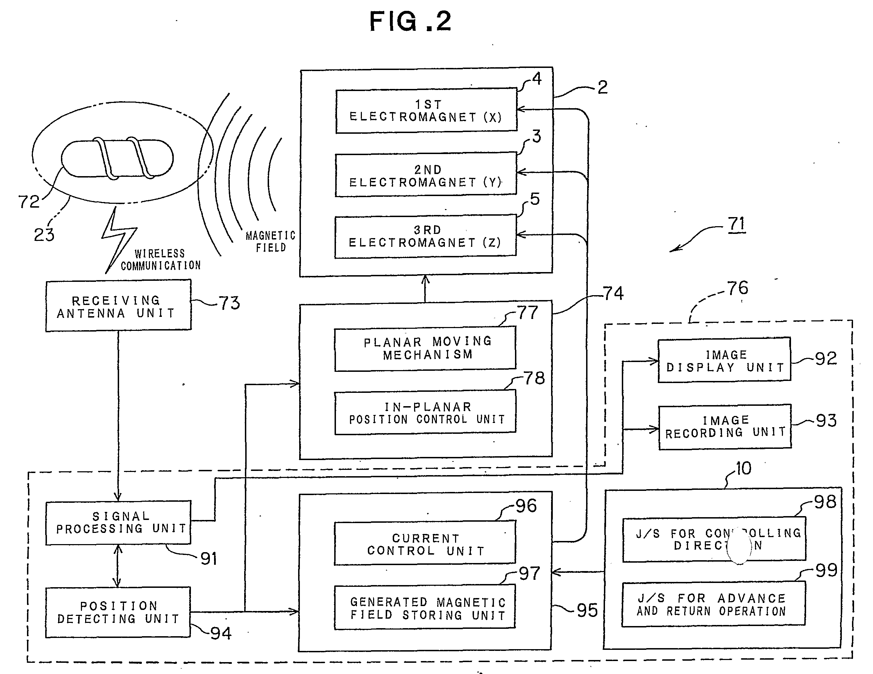

Fig. 2 shows the internal structure of the planar moving mechanism unit 74 and the control unit 76.

Referring to Fig. 2, the capsule medical apparatus 72 modulates image information by picking-up an image of the body cavity, and sends the image by wireless manner to the outside of the body.

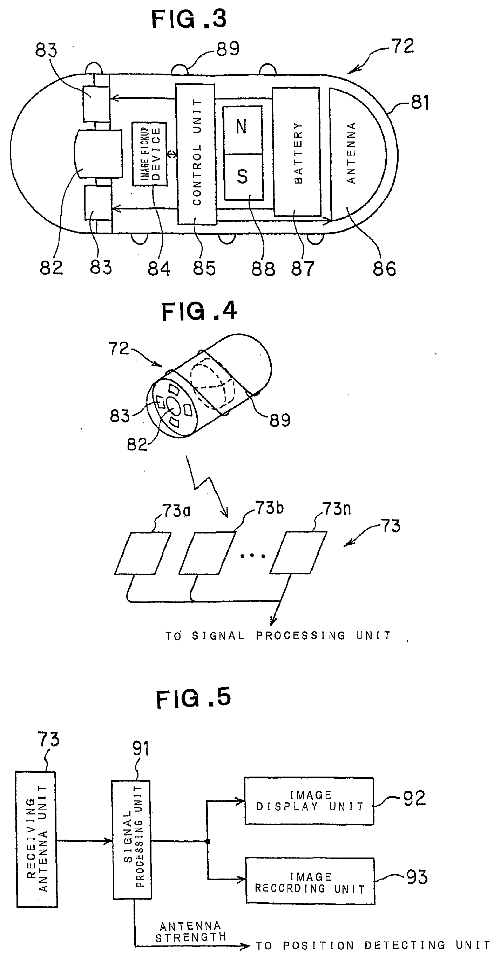

Referring to Fig. 3, the capsule medical apparatus 72 comprises: a capsule container 81, serving as an insertion unit inserted in the body cavity having one-end having a semispherical transparent member; an objective lens 82 arranged near the center of the container 81; and an image pickup device 84, such as a CCD, at the image forming position of the objective lens 82. A plurality of light emitting devices (abbreviated to LEDs) 83, serving as illuminating means, are arranged around the objective lens 82.

A control unit 85 controls the driving operation of the LEDs 83 and the image pickup device 84. The control unit 85 performs the signal processing of the signal picked-up by the image pickup device 84. For example, the control unit 85 compresses the signal, then modulates the signal, and sends the signal by wireless manner via an antenna 86. The container 81 includes: a battery 87 for supplying the power to the control unit 85; and a magnet 88, serving as magnetic-field operated means (magnetic-field operated unit), which operates on the magnetic field generated by the magnetic field generating unit 2 near the center of the container 81 in the longitudinal direction thereof.

Outside the container 81, a spiral structure 89 is arranged to be spirally projected from a cylindrical outer surface of the container 81. The capsule medical apparatus 72 is efficiently propelled by rotating the spiral structure 89 in contact with the inner wall of the body cavity.

The receiving antenna unit 73 shown in Fig. 2 receives a wireless signal, that is, electromagnetic waves, sent from the antenna 86 of the capsule medical apparatus 72. Referring to Fig. 4, the receiving antenna unit 73 comprises a plurality of antennas 73a, 73b, ..., 73n.

Referring to Fig. 2, a signal processing unit 91 receives the signals received by the plurality of antennas 73a, 73b, ..., 73n. Referring to Fig. 2 or 5, the signal processing unit 91 demodulates the signals, an image display unit 92 displays the sent image, and an image recording unit 93 records the image.

The signal processing unit 91 sends, to a position detecting unit (position/posture detecting unit) 94, the signal received by the receiving antenna unit 73 or an antenna strength signal, serving as the strength of the signals received by the receiving antenna unit 73.

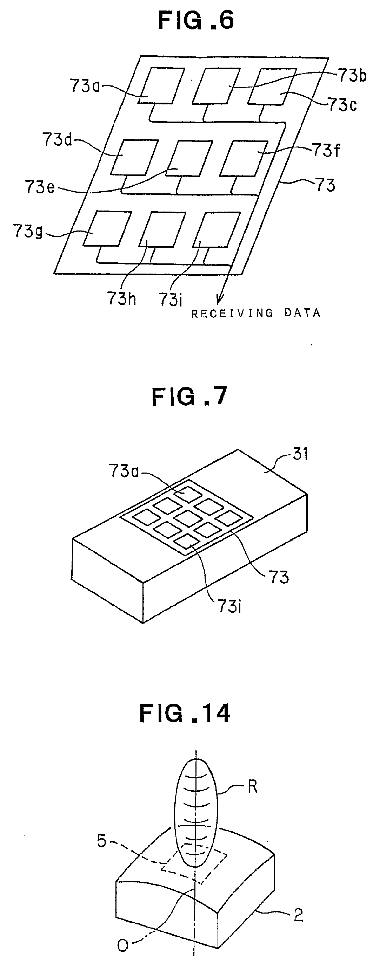

The position detecting unit 94 detects, based on the antenna strength signal, the three-dimensional position and the posture of the capsule medical apparatus 72. In this case, the receiving antenna unit 73 shown in Fig. 4 (arranged on the top of the magnetic field generating unit 2 as shown in Fig. 1) comprises the plurality of antennas 73a, 73b, ..., 73n arranged two-dimensionally as shown in Fig. 6.

In this case, the antenna 73e, serving as one reference, is arranged on a central axis O of the magnetic field generating unit 2, and the plurality of antennas 73a to 77d and 73f to 73i are arranged therearound.

The plurality of antennas 73a to 73i receive the signals sent from the antenna 86 of the capsule medical apparatus 72, and the position of the capsule medical apparatus 72 is detected based on the strength of electromagnetic field (antenna strength signal).

That is, the strength of electromagnetic field of the receiving signal using the positions of the antenna 73j (j = a to i), as the reference, is proportional to the square of the distance. Then, the three-dimensional position of the capsule medical apparatus 72 is calculated by using the trigonometry. Further, the direction of the capsule medical apparatus 72, e.g., the direction of the magnet 88 is detected. An antenna for detecting the image signal from the capsule medical apparatus 72 may be formed, independently of an antenna for detecting the position (and posture).

According to the first embodiment, the position of the capsule medical apparatus 72 is detected by using the electromagnetic field, thereby calculating the position with high precision without interference with the guiding magnetic field. Further, since the electromagnetic field is used to send the image data, both the functions are shared and the efficient use is possible.

The positional information and the posture information (direction information) of the capsule medical apparatus 72 detected by the position detecting unit 94 are sent to the planar moving mechanism unit 74 and the magnetic field control unit 95 as shown in Fig. 2.

The in-planar position control unit 78 of the planar moving mechanism unit 74 controls the two-dimensional position of the planar moving mechanism 77 based on the positional information. That is, the in-planar position control unit 78 controls the planar moving mechanism 77 so that the center of the magnetic field generating unit 2 matches the position on the X coordinate and Y coordinate of the positional information detected by the position detecting unit 94. The generating direction of the rotating magnetic field in the case of applying the rotating magnetic field is controlled based on the posture information of the capsule medical apparatus 72. As mentioned above, the position detecting unit 94 and the in-planar position control unit 78 form a relative-position detecting mechanism which obtains the positional relationship (relative position) between the capsule medical apparatus 72 and the magnetic field generating unit 2. The in-planar position control unit 78 controls the position of the magnetic field generating unit 2 based on the relative position obtained by the relative-position detecting mechanism.

In order for the magnetic field generating unit 2 to generate the magnetic field in the arbitrary direction, the magnetic field control unit 95 comprises: an electromagnet current control unit (abbreviated to a current control unit) 96 for controlling a value of current (level of magnetic field) flowing to electromagnets; and a generated magnetic field storing unit 97 which stores the direction and level of the generated magnetic field. The magnetic field generating unit 2 comprises: a first electromagnet unit having electromagnets 4a and 4b, a second electromagnet unit having electromagnets 3a and 3b, and a third electromagnet unit 5 having the electromagnet 5.

The receiving antenna unit 73 is two-dimensionally moved together with the magnetic field generating unit 2. That is, generally, the planar moving mechanism 77 moves and sets the receiving antenna unit 73 at the position of the antenna 73e, as the reference, so as to maximize the receiving strength.

The approximation is possible when the capsule medical apparatus 72 exists just on the top of the antenna 73e having the largest receiving strength. Thus, since the capsule medical apparatus 72 always exists on the top of the antenna 73e, the image is sent stably and efficiently and the precision for detecting the position is improved. In addition, advantageously, the number of antennas is reduced and the algorithm for controlling the position of the magnetic field generating unit is easy.

The receiving antenna unit 73 is arranged on the top of the magnetic field generating unit 2 as shown in Fig. 1 or the like, thereby being moved together with the magnetic field generating unit 2. Further, referring to Fig. 7, the receiving antenna unit 73 may be attached to the inside of the top of the bed 31 according to a modification.

Referring to Fig. 2, the magnetic field control unit 95 is connected to the instruction/operation unit 10.

Referring to Fig. 1 and an enlarged view thereof, the instruction/operation unit 10 comprises: a joystick 98 for controlling the direction and a joystick 99 for advance and return operation.

Further, the instruction/operation unit 10 comprises a keyboard 100 for setting the magnetic field which sets the direction and the level of the generated magnetic field in accordance with the instructing operation via the magnetic field control unit 95.

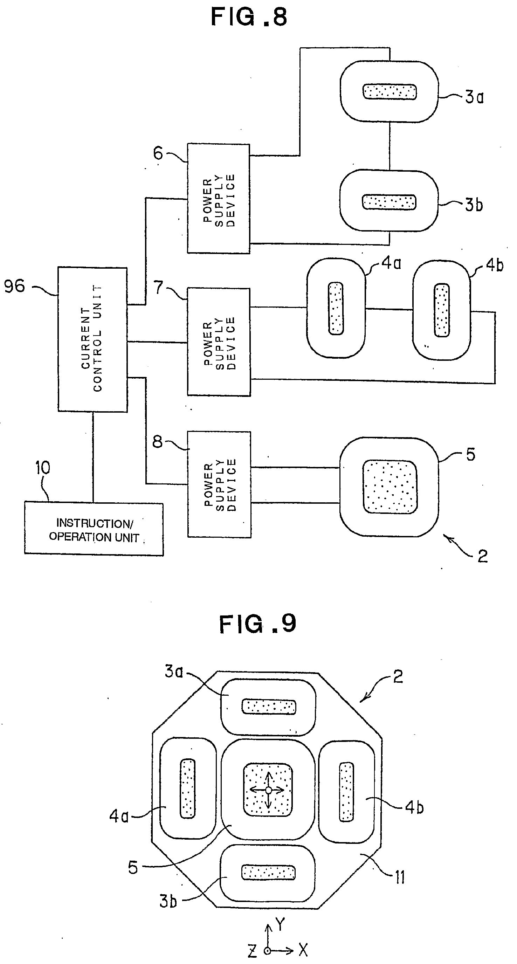

Fig. 8 shows the structure of the magnetic field generating unit 2 and the current control unit 96 which supplies the current for generating the magnetic field to the magnetic field generating unit 2.

Referring to Fig. 8, the current control unit 96 is connected to power supply devices 6 to 8, and controls the magnetic fields generated by the electromagnets 3a and 3b for generating the magnetic field in the up/down direction, by the electromagnets 4a and 4b for generating the magnetic field in the horizontal direction, and by the electromagnet 5 for generating the magnetic field in the vertical direction, forming the magnetic field generating unit 2, to which the current is supplied from the power supply devices 6 to 8. The current control unit 96 may include the power supply devices 6 to 8.

Further, a user controls the generated magnetic field by operating the instruction/operation unit 10 connected to the current control unit 96.

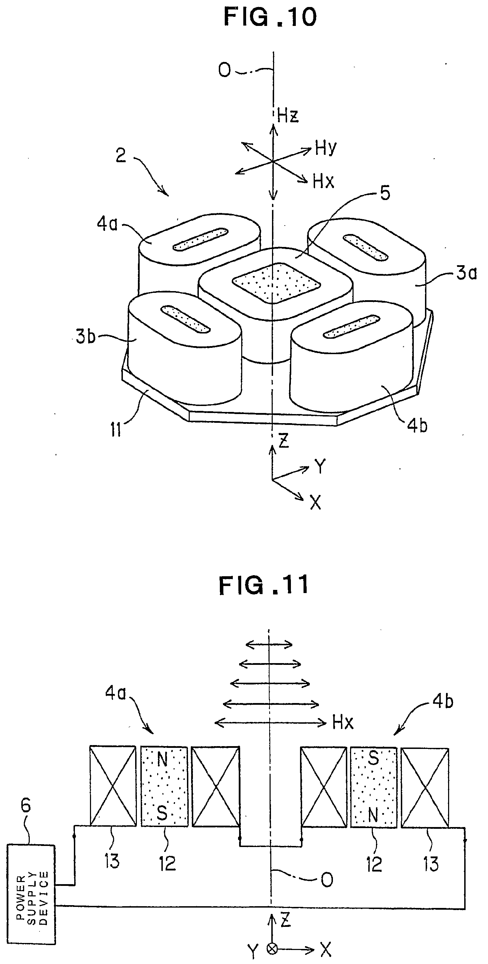

According to the first embodiment, referring to Figs. 9 and 10, the magnetic field generating unit 2 comprises the electromagnets 3a and 3b for generating the magnetic field in the Y direction (up/down direction), the electromagnets 4a and 4b for generating the magnetic field in the X direction (horizontal direction), and the electromagnet 5 for generating the magnetic field in the Z direction (vertical direction), which are symmetrically arranged on the same plane.

The electromagnets 3a and 3b, forming a pair, have the matching characteristics. Preferably, the electromagnets 4a and 4b, forming a pair, have the matching characteristics. Preferably, the electromagnet 3a (3b) and the electromagnet 4a (4b) have the matching characteristics.

In other words, in the case of manufacturing the magnetic field generating unit 2 according to the first embodiment, the electromagnets 3a, 3b, 4a, and 4b, forming the pairs, may have the symmetric arrangement of the same electromagnets. Advantageously, the costs are reduced.

Referring to Fig. 10, assuming that the orthogonal coordinate system of X, Y, and Z is set, the electromagnet 5 for generating the magnetic field in the Z direction (vertical direction) is arranged in the center of a planar base 11, the electromagnets 3a and 3b for generating the magnetic field in the X direction are symmetrically arranged in the X direction (up/down direction) so as to sandwich the electromagnet 5 for generating the magnetic field in the Z direction from the direction orthogonal to the Z axis, and the electromagnets 4a and 4b for generating the magnetic field in the Y direction are symmetrically arranged in the direction orthogonal to the electromagnets 3a and 3b for generating the magnetic field in the X direction, that is, in the Y direction (horizontal direction). According to the first embodiment, the three sets of electromagnets 3a and 3b, 4a and 4b, and 5 have the same height.

Fig. 11 shows the cross-sectional structure of the electromagnets 4a and 4b for generating the magnetic field in the Y direction and the principle diagram of generating the magnetic field.

Referring to Fig. 11, the electromagnets 4a and 4b with the same characteristics are linear-symmetrically arranged on both sides of the central axis O. In this case, coils 13 wound to an iron core portion 12 of quadratic prism containing a ferromagnetic member are respectively formed with the electromagnets 4a and 4b having the same number of wirings each having opposite ones, which are serially connected. Thus, the power supply 6 supplies DC current to the coils 13 of the electromagnets 4a and 4b. Thus, the electromagnets 4a and 4b are magnetized in the opposite direction, and have the equal strength of magnetic field.

Referring to Fig. 11, one electromagnet 4a is magnetized to the poles N and S in the Z direction in parallel with the central line 0, and the other electromagnet 4b is magnetized to the poles S and N. Therefore, at the position on the top of the height position of one of the magnetic poles of the electromagnets 4a and 4b on the central line O, a magnetic field Hx is generated in the vertical direction to the central axis O and in the arrangement direction of the electromagnets 4a and 4b, that is, in the horizontal direction (X direction).

As shown by the length of an arrow in Fig. 11, the level of the magnetic field Hx on the central axis O is reduced as the distance (height) of the top end to both the magnetic poles N and S is longer. However, since both the electromagnets 4a and 4b have the same characteristics and are linear-symmetrically arranged to the central axis O, the level of the magnetic field Hx on the central axis O changes to be reduced depending on the increase in distance. However, the direction of the magnetic field Hx on the central axis O does not change.

Referring to Fig. 10, in the case of arranging the electromagnets 3a, 3b, 4a, 4b, and 5, a magnetic field Hy in the Y direction is generated by the electromagnets 3a and 3b on the Z axis, serving as the central axis O, the magnetic field Hx in the X direction is generated by the electromagnets 4a and 4b, and a magnetic field Hz in the Z direction is generated by the electromagnet 5, as shown in Fig. 10.

The operation (of the keyboard 100) of the instruction/operation unit 10 shown in Fig. 8 varies the polarity and the value of DC current supplied to the electromagnets 3a, 3b, 4a, 4b, and 5 by the power supply devices 6 to 8, thereby arbitrarily setting the level and the direction of the magnetic field generated on the top position of the electromagnets 3a, 3b, 4a, 4b, and 5 (electromagnet 5 when the heights of the electromagnets are equal) on the Z axis. That is, the three-dimensional magnetic field is generated in the arbitrary direction with the arbitrary level on the top position of the electromagnet 5.

As mentioned above, according to the first embodiment, the three sets of the electromagnets 3a and 3b, 4a and 4b, and 5 are set on the plane, thereby generating the three-dimensional magnetic field on the space on the top of the electromagnets 3a, 3b, 4a, 4b, and 5 on the central axis.

That is, to the position or space to which the three-dimensional magnetic field is applied, the magnetic field generating unit 2 is approached in the arbitrary direction in the space under the control operation of the planar moving mechanism unit 74 and is arranged near the place or space, thereby generating the three-dimensional magnetic field in the space.

The current control unit 96 has, as calibration data, the strength of magnetic field generated in the directions (X, Y, and Z directions) per 1A at the positions (heights) on the central axis O.

Further, the user's operation of the instruction/operation unit 10 controls the current flowing to the electromagnets 3a, 3b, 4a, 4b, and 5 from the power supply devices 6 to 8 to be DC current, vibrating current, and rotating current (due to the vibrating current with the phase difference), thereby generating the static magnetic field, vibrating magnetic field, and rotating magnetic field.

According to the first embodiment, advantageously, the position or space to which the magnetic field is applied is easily approached, thereby arbitrarily applying (generating) the three-dimensional magnetic field with high precision. In particular, in the case of generating the magnetic field with the large amount of change in magnetic field in the axial directions, such as the rotating magnetic field and the vibrating magnetic field in the general medical apparatus with a low position-moving-speed, the amount of movement of the medical apparatus is small without varying the position/posture of the magnetic field generating unit depending on the direction of the generated magnetic field. Thus, the driving speed of the position/posture varying unit is low. Advantageously, the medical apparatus is stably controlled, the size of the position/posture varying unit is reduced, the power consumption is low, the structure is simplified, and the magnetic guiding medical system has the efficient structure.

Further, the electromagnets are arranged on the plane and the magnetic field generating unit is moved only on the plane of the arrangement of the electromagnets. When the living body approaches the space for generating the magnetic field, there is no interference of the magnetic field generating unit or moving mechanism with the living body. Therefore, the moving mechanism is easily controlled and, advantageously, the controllability and stability are improved. Further, the magnetic field generating unit having heavy weight is set under the bed and the center of gravity of the overall apparatus is reduced. Thus, the mechanical stability is improved.

The electromagnets 3a, 3b, 4a, 4b, and 5 are collected in one direction. Near the central axis 0, as the electromagnets 3a, 3b, 4a, 4b, and 5 are far from the pole face, the entire generated magnetic fields (Hx, Hy, Hz) in the directions are reduced. Near the central axis O of the electromagnet 5, the direction of the magnetic field does not greatly change (if the strength changes, the magnetic field whose direction does not change is generated.

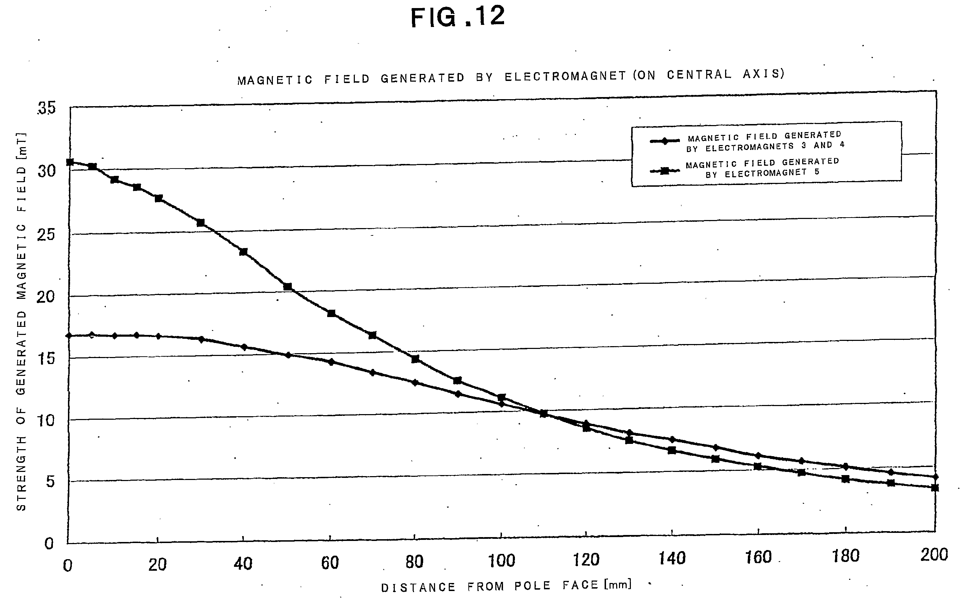

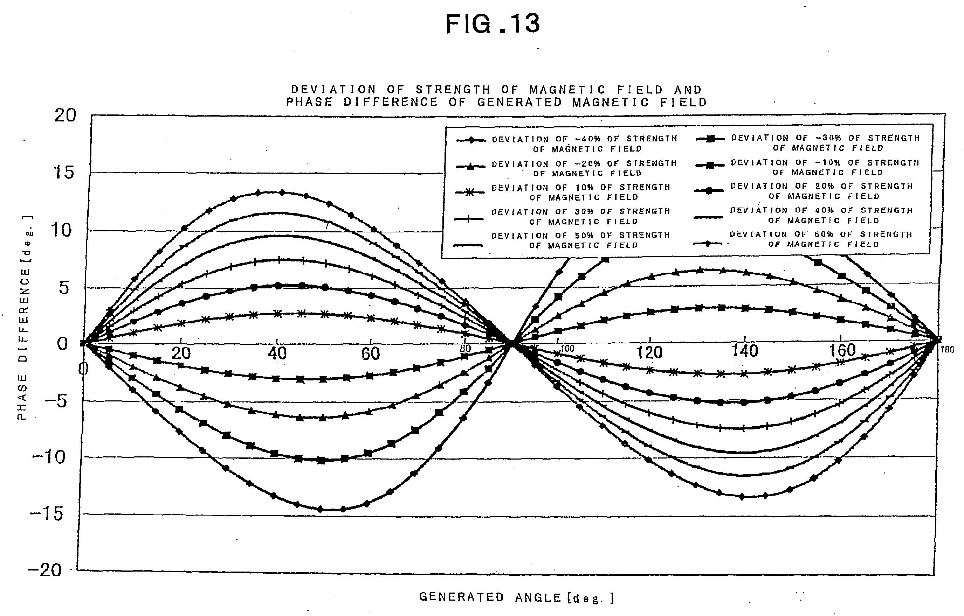

Referring to Figs. 9 and 10, the magnetic fields of the electromagnets 3a, 3b, 4a, 4b, and 5 generated by the magnetic field generating unit 2 are measured. Figs. 12 and 13 show measurement results.

Fig. 12 shows the measurement results of magnetic fields generated by the electromagnets 3a, 3b, 4a, 4b, and 5 on the central axis O relative to the distance from the pole face (specifically, the magnetic-field surface (specifically, the pole face on the top of the electromagnet 5). Referring to Fig. 12, the electromagnets 3a and 3b are abbreviated to the electromagnet 3, and the electromagnets 4a and 4b are abbreviated to the electromagnet 4. The electromagnets 3a and 3b and the electromagnets 4a and 4b have the same characteristics, and have different arrangement directions. Therefore, the electromagnets 3a and 3b and the electromagnets 4a and 4b have the same graph.

Based on the characteristics, the electromagnets 3a and 3b, the electromagnets 4a and 4b, and the electromagnet 5 have different strengths of magnetic fields at the distance near the pole face. However, when the distance is longer to some degree, the electromagnets 3a and 3b, the electromagnets 4a and 4b, and the electromagnet 5 have the same characteristics of the same strength.

Fig. 13 shows the influence on the deviation from the original direction of the magnetic field due to the deviation of the strength of one magnetic field from the instructed value in the electromagnets having the characteristics shown in Fig. 12 (two sets of the electromagnets 3a and 3b and the electromagnets 4a and 4, and the electromagnet 5 having the same characteristics). That is, it is indicated, by the angle (angle difference), how much the deviation of strength of generated magnetic field influences on the deviation in direction of the magnetic field.

Based on the result shown in Fig. 13, it is understood that, even if the strength of the magnetic field is excessively different from the instructed strength of magnetic field, the angle deviated from the direction of the generating magnetic field is kept to be relatively small.

When the deviation of the angle from the instructed target magnetic field to be generated is allowable up to 10 [deg.], the difference in strengths of magnetic field generated in the directions is allowable up to 40%. Therefore, even if being used in a state that the generated magnetic field is roughly controlled with the simple control operation, the magnetic field generating unit 2 can generate the three-dimensional magnetic field under the wide allowable range.

According to the first embodiment, the position of the magnetic field generating unit 2 is moved based on the positional information detected by the position detecting unit 94 as mentioned above, and it is controlled that the magnetic field generating unit 2 always exists substantially directly below the capsule medical apparatus 72 in the body cavity of the patient 23. In other words, referring to Fig. 14, it is controlled that the capsule medical apparatus 72 is positioned within a guidable region R near just above the magnetic field generating unit 2.

As mentioned above, the capsule medical apparatus 72 having the insertion unit inserted in the body cavity is controlled to generate the optimum magnetic field near the position of the capsule medical apparatus 72.

In this state, the magnetic field generating unit 2 applies the rotating magnetic field to the capsule medical apparatus 72, thereby efficiently propelling forward the capsule medical apparatus 72 and returning it if necessary.

Fig. 14 schematically shows the guidable region R that can be magnetically guided by the magnetic field by the magnetic field generating unit 2. The guidable region R corresponds to approximately a cylindrical or oval portion along the direction 0 of the magnetic field by the third electromagnet unit 5 in the magnetic field generating unit 2.

As mentioned above, since the guidable region R is limited to a part of region on the top of the magnetic field generating unit 2, the position of the magnetic field generating unit 2 is controlled based on the positional information of the position detecting unit 94 so that the capsule medical apparatus 72 is within the guidable reign R.

According to the first embodiment, it is controlled that the capsule medical apparatus 72 is within the guidable region R near just on the top of the third electromagnet 5 unit in the magnetic field generating unit 2. In this state, the magnetic field generating unit 2 applies the rotating magnetic field to the capsule medical apparatus 72.

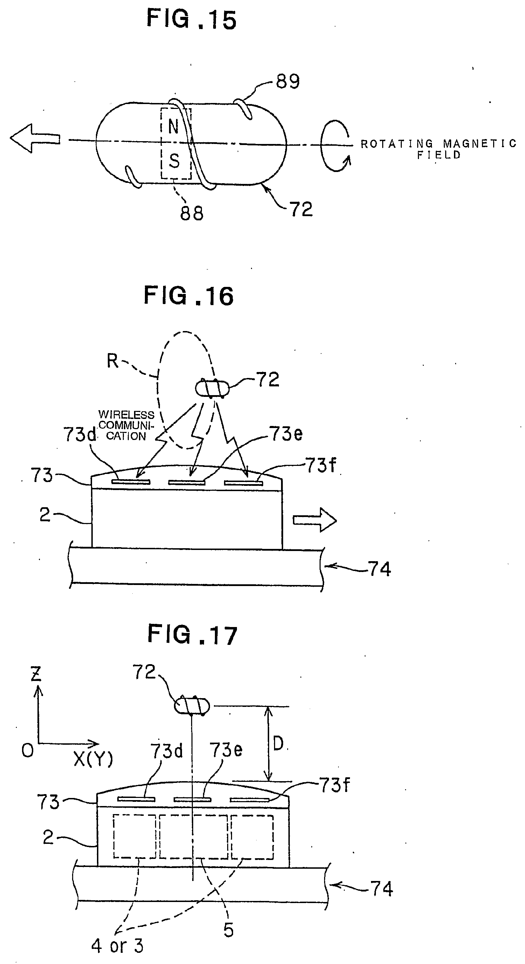

Referring to Fig. 15, the rotating magnetic field is applied to the capsule medical apparatus 72 and, thus, the spiral structure 89 converts the rotation to propelling force. Then, the direction of the generating surface of the rotating magnetic field controls the propulsion in the propelling direction of the capsule medical apparatus 72, that is, to the forward or backward.

The following control operation is considered as an example upon controlling the direction and level of the precisely-generated magnetic field.

As mentioned above, although the difference (deviation in the direction of magnetic field) in the direction of the magnetic field actually-generated from the target direction of the magnetic field is small, the rotating magnetic field is generated, then, the above-mentioned deviation in the direction of the magnetic field is observed as the rotating deviation of the rotation of the capsule medical apparatus 72.

Upon rotating and moving the capsule medical apparatus 72 by applying the rotating magnetic field, the signal processing unit 91 outside the body calculates a rotating-angle speed of the capsule medical apparatus 72 from the continuously obtained images by pattern matching. The rotating-angle speed of the image is compared with the rotating-angle speed of the rotating magnetic field to be generated, and the deviation is calculated between the direction of the magnetic field to be generated and the direction of the magnetic field that is actually generated.

The obtained amount of deviation is fed-back and then the current flowing to the electromagnets 3, 4, and 5 of the magnetic field generating unit 2 is controlled. The rotating-angle speed of the capsule medical apparatus 72 is calculated based on the obtained image. Therefore, the magnetic sensor for detecting the generated magnetic field does not need to be arranged to the capsule medical apparatus 72. Thus, the magnetic field is generated with high precision, and the capsule medical apparatus 72 is smoothly controlled.

Fig. 16 shows the state in which the planar moving mechanism 77 moves the magnetic field generating unit 2 on the two-dimensional surface so that the capsule medical apparatus 72 is within the guidable region R as shown in Fig. 14.

That is, the antenna 73j (j = a to i) of the receiving antenna unit 73 receives the signal sent by wireless manner from the capsule medical apparatus 72, and the position of the capsule medical apparatus 72 is detected based on the strength of electromagnetic field (abbreviated to an antenna strength) received by the antenna 73j. The planar moving mechanism 77 moves the magnetic field generating unit 2 based on the positional information so that the capsule medical apparatus 72 is within the guidable reign R.

In the case shown in Fig. 16, it is detected that the capsule medical apparatus 72 is slightly deviated to the right side from the guidable region R and therefore it is controlled based on the positional information so that the magnetic field generating unit 2 is moved to the right.

Referring to Fig. 17, the capsule medical apparatus 72 is kept just on the top of the third electromagnet 5.

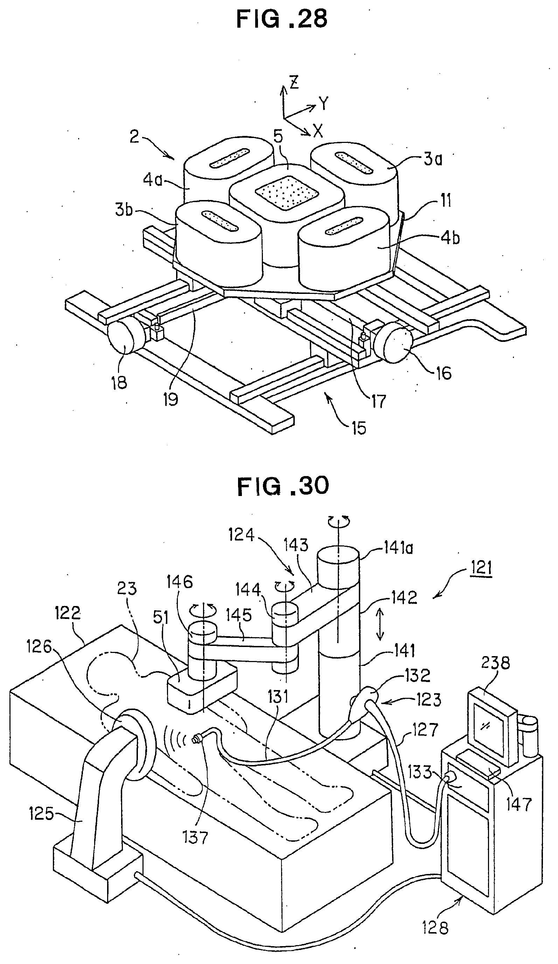

Fig. 28 shows the structure of a parallel-moving mechanism 15 that is moved in parallel on the plane by the planar moving mechanism 77 by attaching the magnetic field generating unit 2 to the top of the planar moving mechanism 77.

Referring to Fig. 28, the parallel-moving mechanism 15 moves and sets the base 11 at an arbitrary two-dimensional position. In the example shown in Fig. 28, the rotation of the motor 16 is driven, thereby moving the base 11 in the X direction with the rotation of a ball screw 17 or the like. The rotation of a motor 18 is driven, thereby moving the base 11 in the Y direction with the rotation of a ball screw 19. That is, the rotation of the motors 16 and 18 is driven, thereby setting the magnetic field generating unit 2 attached to the base 11 at an arbitrary two-dimensional position.

Moving means of the height direction is arranged, thereby setting the magnetic field generating unit 2 at an arbitrary three-dimensional position.

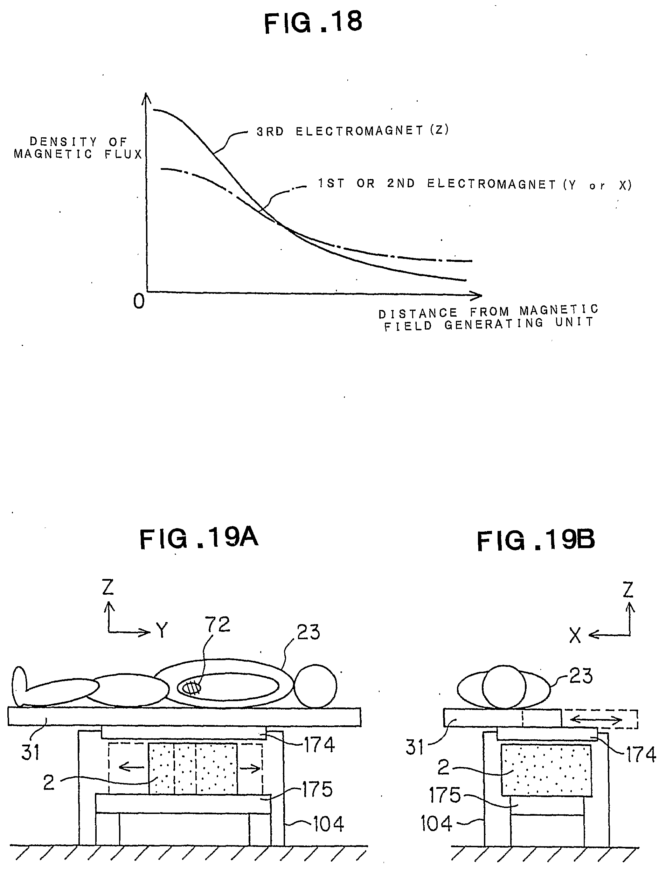

In the state shown in Fig. 17, it is possible to obtain necessary information for generating the level and the direction of the optimum magnetic field in the case of moving the capsule medical apparatus 72 in the arbitrary direction at the current position of the capsule medical apparatus 72, based on information on a distance D form the top surface of the magnetic field generating unit 2 or the receiving antenna unit 73 to the capsule medical apparatus 72 and data on the generated magnetic field per unit current of the electromagnet shown in Fig. 18 stored in a memory unit of the generated magnetic field storing unit 97 shown in Fig. 2.

That is, it is possible to obtain the distance from the capsule medical apparatus 72 to the electromagnets 3 to 5 based on the current position of the capsule medical apparatus 72. Further, it is possible to calculate the values of current flowing to the electromagnet 3 to 5 upon generating the magnetic field in the arbitrary direction based on the data (shown in Fig. 18) on the generated magnetic field per unit current at the obtained distance.

The component of the magnetic field in the Z direction is calculated based on the direction and the level of the magnetic field to be generated, and the current flowing to the electromagnet 5 is determined by referring to the data on the generated magnetic field per unit current. Similarly, with respect to the X direction and the Y direction, the current flowing to the electromagnets 3 and 4 is determined.

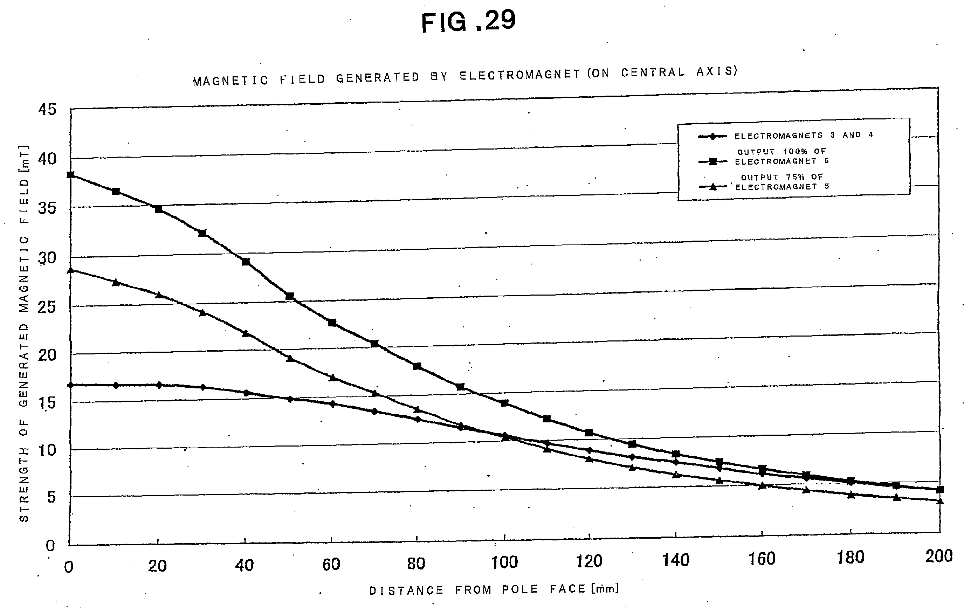

Fig. 29 shows the measurement result of the generated magnetic field on the central axis O in the case of changing the value of the current flowing to the central electromagnet 5.

As will be understood with reference to Fig. 29, when the distance D is long, the value of the current flowing the central electromagnet 5 is increased depending on the ratio between the component of the magnetic field in the vertical direction and the component of the magnetic field in the horizontal direction. Thereby, the deviation of the direction of the generated magnetic field is reduced at the distance far from the pole face. On the contrary, when the distance D is short, the value of the current flowing to the central electromagnet 5 is reduced depending on the ratio between the component of the magnetic field in the vertical direction and the component of the magnetic field in the horizontal direction, thereby reducing the deviation of the generated magnetic field at the near-distance side.

Further, the value of the current flowing to the peripheral electromagnets 3a and 3b and electromagnets 4a and 4b may be changed, thereby controlling the generated magnetic field.

As mentioned above, the current of the electromagnet is determined only by the distance D (Z coordinate), and only the magnetic field data on the central axis O may be stored. Therefore, the amount of data stored in the memory unit is reduced. Advantageously, the control algorithm is simplified, the structure is easy, and controllability is stable.

Further, upon generating the magnetic field of a repeating pattern of the rotating magnetic field or vibrating magnetic field, only the maximum value (amplitude) of a current pattern of the electromagnet may be varied depending on the distance D. Therefore, advantageously, the system and the control algorithm are further simplified.

In this case, the capsule medical apparatus 72 is held just on the top of the electromagnet 5 as mentioned above. The distance D between the magnetic field generating unit 2 and the capsule medical apparatus 72 in this case is approximately minimum. Therefore, the magnetic field generated by the magnetic field generating unit 2 on the near distance side is efficiently used, and the capsule medical apparatus 72 can be guided in the region having the matching magnetic field.

Further, when the capsule medical apparatus 72 exists near the magnetic field generating unit 2 and the strength of magnetic field is sufficiently ensured, the upper limit of the strength of the generated magnetic field may be provided. Thus, the power consumption is realized because unnecessary current does not need to flow to the electromagnet.

According to the first embodiment, the receiving antenna unit 73 having a plurality of antennas receives the wireless signals from the capsule medical apparatus 72, and the position of the capsule medical apparatus 72 is calculated based on the antenna strength signal in this case.

The guidable region R is ensured by controlling the current flowing to the electromagnets of the magnetic field generating unit 2 with the positional information, and by moving the magnetic field generating unit 2, the capsule medical apparatus 72 is controlled such that the magnetic field generated by the magnetic field generating unit 2 is set within the guidable region R for magnetic guiding operation. When the rotating magnetic field is applied to the capsule medical apparatus 72 in this state and the capsule medical apparatus 72 is thus moved at the position within the wide range in the body cavity, the relative position between the capsule medical apparatus 72 and the magnetic field generating unit 2 are controlled, thereby holding the capsule medical apparatus 72 at the position for continuously easy guiding operation. Thus, the capsule medical apparatus 72 is magnetically propelled with high smoothness.

Upon applying the rotating magnetic field to the capsule medical apparatus 72 by the joystick 98 for controlling the direction and the joystick 99 for advance and return operation shown in Fig. 1, the capsule medical apparatus 72 is rotated. Thus, the position detecting unit 94 detects the positional information and further detects the posture of the capsule medical apparatus 72, and the signal processing unit 91 performs, based on the detected signals, the processing for rotating the image received form the capsule medical apparatus 72 in the inverse direction of the rotation, and generates a still image.

Then, the image display unit 92 displays the still image.

Upon rotating the capsule medical apparatus 72, the image display unit 92 stops the rotation and displays the image. Then, the user views the image that does not have the image rotation due to the rotation of the capsule medical apparatus 72 displayed on the image display unit 92 and instructs the directional change to the arbitrary directions of vertical and horizontal directions by operating the joystick 98 for controlling the direction.

The propulsion to the forward or backward on the image is instructed by operating the joystick 99 for advance and return operation.

In the case of instructing the propulsion by the joystick 99 for advance and return operation and the spiral structure 89 arranged on the outer-circumferential surface of the capsule medical apparatus 72 is right-spirally arranged, and the joystick 99 for advance and return operation is inclined in the upper direction, the rotating magnetic field is generated in the right rotating direction relative to the front direction on the screen, thereby moving the capsule medical apparatus 72 forward on the screen.

Further, upon inclining downward the joystick 99 for advance and return operation, the rotating magnetic field is generated in the left rotating direction relative to the front direction on the screen, thereby moving backward the capsule medical apparatus 72 on the screen.

According to the first embodiment, upon moving the position of the capsule medical apparatus 72 within the wide range in the body cavity, the relative position between the capsule medical apparatus 72 and the magnetic field generating unit 2 is controlled, thereby continuously holding them at the position for easy magnetic guiding operation and magnetically propelling the capsule medical apparatus 72.

According to the first embodiment, the planar moving mechanism unit 74 has the planar moving mechanism 77 that moves the magnetic field generating unit 2 in the X and Y directions as shown in Fig. 1. In place of this, referring to Figs. 19A and 19B, the planar moving mechanism unit 74 may have a bed horizontal moving mechanism 174 that moves the bed 31 in the X direction and a Y-direction-moving mechanism 175 that moves the magnetic field generating unit 2 only in the Y direction according to a first modification.

Referring to Fig. 19B, the bed 31 is moved in the X direction by the bed horizontal moving mechanism 174 arranged on the top of a bed supporting base 104.

Referring to Fig. 19A, the Y-direction-moving mechanism 175 is arranged in the bed supporting base 104, and the magnetic field generating unit 2 arranged on the top surface of the Y-direction-moving mechanism 175 is moved only in the Y direction by the Y-direction-moving mechanism 175.

With the above-mentioned structure, the following advantages are obtained.

That is, upon increasing the width of the magnetic field generating unit 2 relative to the width of the bed 31, the magnetic field generating unit 2 is moved only in the longitudinal direction relative to the rectangular bed 31. Therefore, the lateral width of the device is reduced. Further, since the number of driving axes of the magnetic field generating unit 2 having the heavy weight and the large number of wirings is reduced. Thus, the driving part is simple and the overall device is reduced in size and weight, and the efficiency is improved.

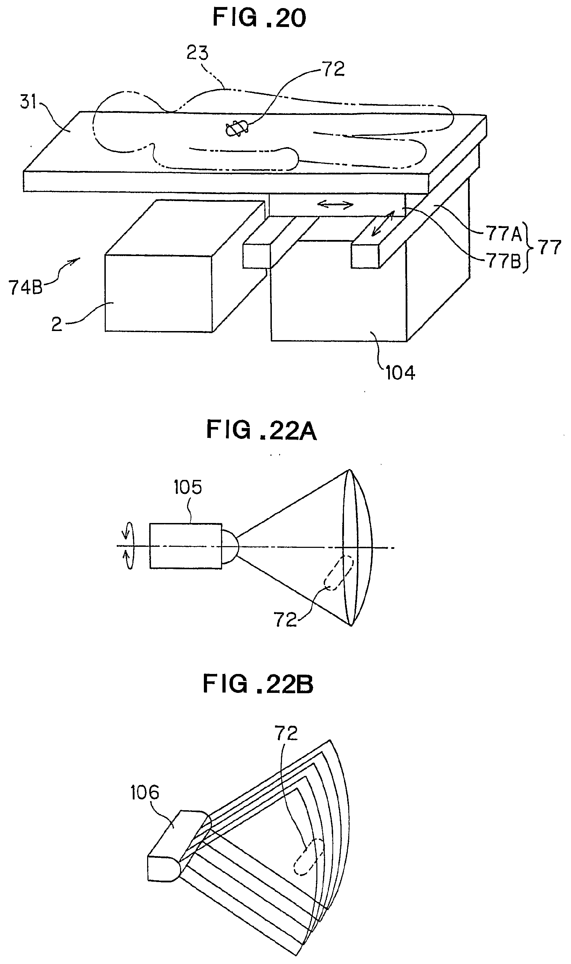

Fig. 20 shows the structure of a planar moving mechanism unit 74B according to a second modification. As mentioned above, in the planar moving mechanism unit 74 shown in Figs. 1 and 2, the magnetic field generating unit 2 is moved based on the positional information of the capsule medical apparatus 72. However, according to the second modification, the bed 31 is moved based on the positional information.

That is, the planar moving mechanism 77 arranged on the top surface of the bed supporting base 104 supports the main body of the bed 31 on which the patient 23 is laid. In this case, the magnetic field generating unit 2 is arranged under the bottom side of the bed 31. In this case, since the magnetic field generating unit having the heavy weight and the large number of wirings is fixed, the structure of the driving portion is simple. Thus, the overall device is reduced in size and weight and the efficiency is improved. In particular, upon increasing the sum of the moving range and the width of the magnetic field generating unit relative to the width of bed, the lateral width of the device is reduced.

According to the first embodiment, the planar moving mechanism unit 74 has been used to move the magnetic field generating unit 2 in X and Y directions (to change the area of generated magnetic field above the bed in X and Y directions). In place of this, a tilt moving mechanism can be used (can be replaced with the planar moving mechanism unit 74). The tilt moving mechanism inclines the magnetic field generating unit 2 to change the direction of generated magnetic field. By changing the tilting angle of the magnetic field generating unit 2, the area of magnetic field generated above the bed can be changed to cover the whole guiding area. The tilt moving mechanism can be tilted in two degrees of freedom (i.e. spherically), thus enable to change the area of generated magnetic field above the bed in X and Y directions. The tilt moving mechanism can also be one degree of freedom type (tilts only in a plane) and combined with bed horizontal moving mechanism to achieve X and Y direction movement of the area of generated magnetic field. By only tilting the magnetic field generating unit 2, the space for moving the magnetic field generating unit 2 can be reduced.

According to the embodiment and the like, the position detecting means detects the position by using the signal sent by wireless manner from the capsule medical apparatus 72. However, the positional information may be obtained by using ultrasonic waves as will be described later.

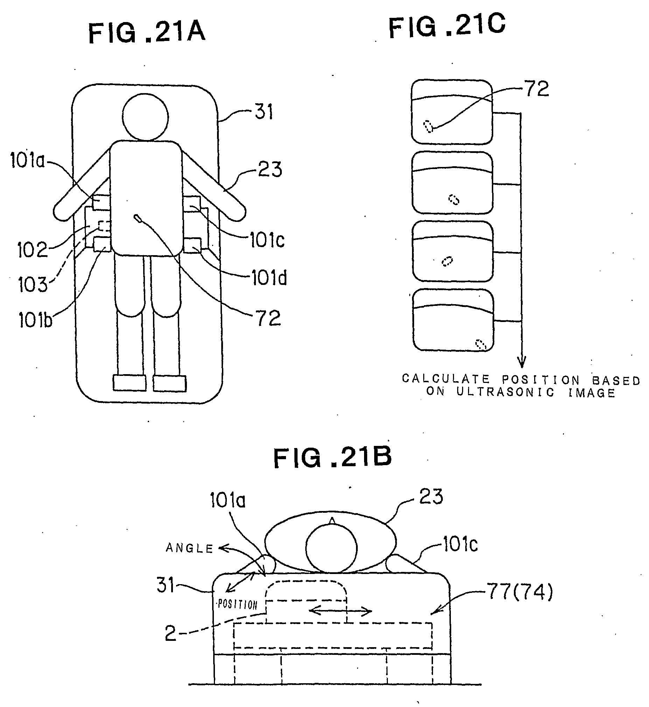

Referring to Figs. 21A and 21B, near the side of the body surface of the patient 23 on the bed 31, a plurality of ultrasonic probes 101a, 101b, ... are arranged, and the distal-end surfaces for receiving and sending the ultrasonic waves of the plurality of ultrasonic probes 101a, 101b, ... come into contact with the body surface of the side of the patient 23.

The plurality of ultrasonic probes 101a, 101b, ... adjust the position and angle for the contact state to the body surface of the patient 23 by an adjusting device 102. The adjusting device 102 has a sensor 103 that detects the information on the position and the angle for the contact state to the patient 23 of the plurality of ultrasonic probes 101a, 101b, .... The sensor 103 comprises an encoder and a linear encoder to detect the contact position and the angle to the patient 23 of the probes 101a, 101b, ....

The sensor 103 outputs the detected information to the planar moving mechanism unit 74 and the magnetic field control unit 95 shown in Fig. 2, and the adjusting device 102 controls the feedback operation of the contact position and angle of the patient 23 by the plurality of ultrasonic probes 101a, 101b, ....

The ultrasonic images obtained by the plurality of ultrasonic probes 101a, 101b, ... are as shown in Fig. 21C. The ultrasonic images of the capsule medical apparatus 72 are extracted, and the position of the capsule medical apparatus 72 on the coordinate system of the bed 31 is calculated based on the plurality of ultrasonic image.

The information on the calculated position of the capsule medical apparatus 72 obtained from the plurality of ultrasonic images are outputted to the planar moving mechanism unit 74 and the magnetic field control unit 95 shown in Fig. 2, and is used for the positional control operation of the magnetic field generating unit 2 and the control operation of the generated magnetic field.

In place of using the plurality of ultrasonic probes 101a, 101b, ..., ultrasonic probes 105 and 106 may be used as shown in Fig. 22A.

Referring to Fig. 22A, the ultrasonic probe 105 is rotated, thereby obtaining information on the three-dimensional ultrasonic waves. Then, the position of the capsule medical apparatus 72 is calculated based on the information on the three-dimensional ultrasonic waves obtained by the ultrasonic probe 105. Further, the calculated information on the position is used.

Referring to Fig. 22B, the array ultrasonic probe 106 is used and the information on the three-dimensional ultrasonic waves is obtained. Then, the position of the capsule medical apparatus 72 is calculated based on the information on the three-dimensional ultrasonic waves. Further, the calculated information on the position is used.

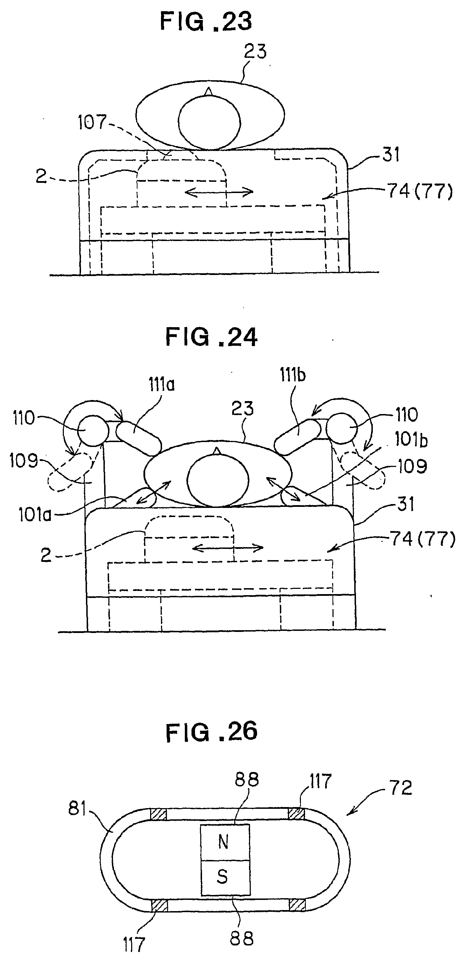

The structure shown in Fig. 23 may be used. Referring to Fig. 23, a planar moving mechanism unit 74C has an ultrasonic probe 107 on the top of the receiving antenna unit 73 arranged to the top of the magnetic field generating unit 2 as shown in Fig. 21B. The planar moving mechanism 77 freely moves the planar moving mechanism unit 74C.

In this case, the moving range for moving the ultrasonic probe 107 on the bottom of the bed 31 is cut-off, thereby keeping the state in which the top of the ultrasonic probe 107 is in contact with the back of the patient 23.

Further, the position of the capsule medical apparatus 72 is calculated based on the ultrasonic image obtained by the ultrasonic probe 107, and the calculated information on the position is used.

Referring to Fig. 24, in addition to the ultrasonic probes 101a, 101b, ... that are in contact with the patient 23 at the position slightly near the back rather than the side thereof as shown in Fig. 21B, a rotating member 110 at the top end of an attaching base 109 that stands on the top of the patient 23 has ultrasonic probes 111a and 111b that are rotatable. The ultrasonic probes 111a and 111b are fixed in contact with the position slightly near the top surface (front surface) from the side surface of the patient 23.

The rotating member 110 is rotated, thereby freely opening and closing the ultrasonic probes 111a and 111b that are in contact with the patient 23 and are apart from the patient.

The ultrasonic frequencies of the ultrasonic probes 101a may be varied.

The plurality of ultrasonic probes 101a may be sequentially driven, thereby obtaining the information on the ultrasonic image.

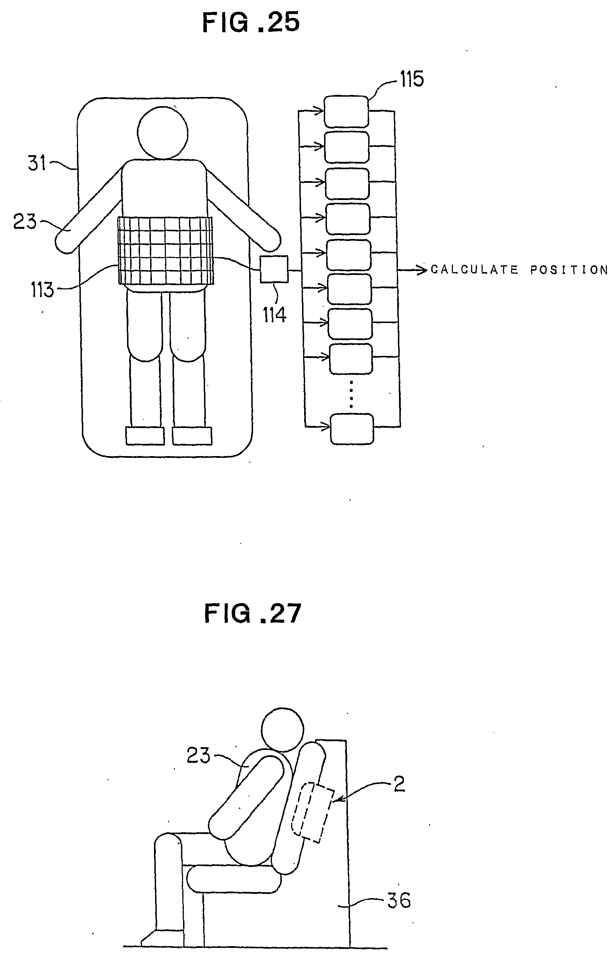

Referring to Fig. 25, an ultrasonic probe array 113 arranged along the cylindrical surface is set to cover the belly of the patient 23.

Then, an ultrasonic image obtained by the ultrasonic probe array 113 is sequentially outputted to the ultrasonic image display device 115 via an ultrasonic observing device 114 for processing the signals of the ultrasonic probe array 113. Further, the position of the capsule medical apparatus 72 may be calculated from the ultrasonic image and the calculated information on the position may be used.

In the case of detecting the positional information from the image by using the ultrasonic probes 101a, the container 81 of the capsule medical apparatus 72 shown in Fig. 26 may have a material 117 for reflecting the ultrasonic waves.

Thus, the position of the capsule medical apparatus 72 is easily calculated from the ultrasonic image.

Although the magnetic field generating unit 2 is arranged in the bed 31, as shown in Fig. 27, the magnetic field generating unit 2 may be arranged in a chair 36.

### (Second arrangement)

Next, a description is given of a second arrangement not according to the present invention with reference to Fig. 30. Fig. 30 shows a magnetic guiding medical system 121 according to the second arrangement.

According to the second arrangement, a magnetic field generating unit 51 for extracorporeally guiding the patient 23 applies the static magnetic field to a magnet 137 arranged to the endoscope 123 inserted in the patient 23, thereby changing the direction of the magnet 137 in the endoscope 123. Therefore, the user, such as an operator, controls the static magnetic field generated by the magnetic field generating unit 51 and changes the direction of the magnet 137 in the desired direction, thereby controlling the direction of the endoscope 123.

The magnetic guiding medical system 121 comprises: the endoscope 123 inserted in the body of the patient 23 placed on a bed 122; a robot arm 124 arranged on one side-surface of the bed 122; the magnetic field generating unit 51 attached to the robot arm 124; and a magnetic sensor 126 arranged on a sensor holding base 125 arranged on the other side surface of the bed 122.

A universal cable 127 of the endoscope 123 is connected to the magnetic guiding medical system 121, and comprises the robot arm 124, the holding base 125, and a control unit 128 connected to the robot arm 124 and the holding base 125 via cables.

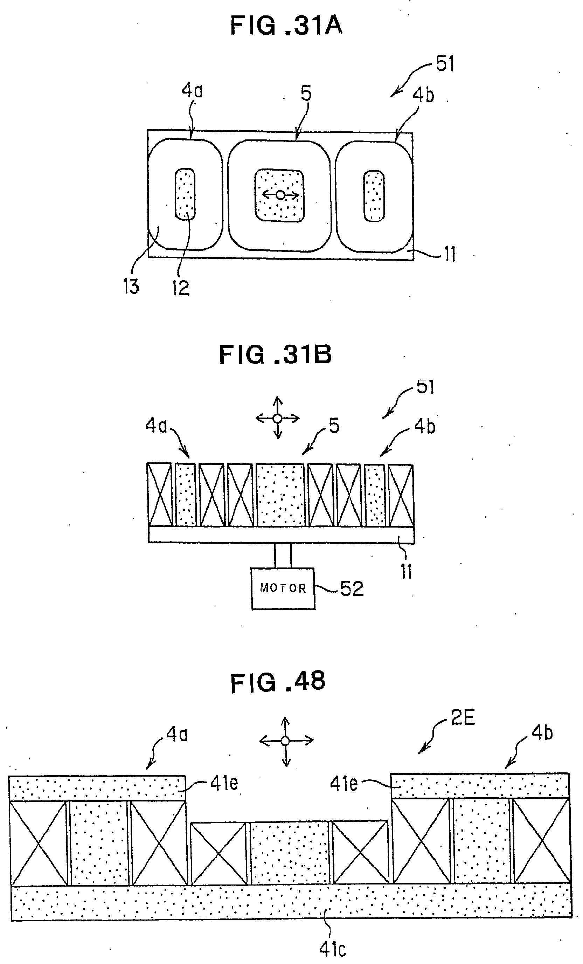

According to the first embodiment, the magnetic field generating unit 2 comprises the three sets of electromagnets. However, according to the second arrangement, referring to Figs. 31A and 31B, the two-dimensional magnetic field generating unit 51 comprises two sets of electromagnets, and generates the two-dimensional magnetic field by the two sets of electromagnets. Incidentally, Fig. 31A is a plan view, and Fig. 31B is a sectional view.

A motor 52 rotates the base 11 having the above components around the central axis O of the central electromagnet 5 if necessary, thereby providing a function of a three-dimensional magnetic field generating unit for generating the three-dimensional magnetic field.

Preferably, the motor 52 for driving the rotation is an electromagnetic motor to which the magnetic shield is applied, or a motor (ultrasonic motor, etc.) that is not influenced from the magnetic power.

Irrespective of the rotation, similarly to the first embodiment, the electromagnet is plain-arranged and the magnetic field generating unit is moved only on the plane having the electromagnet. Therefore, when the living body is close to the space for generating the magnetic field, there is no danger of interference between the magnetic field generating unit and the moving mechanism with the living body. Thus, the moving mechanism is easily controlled and, advantageously, the controllability and the stability are improved. Further, the magnetic field generating unit having large weight is arranged under the bed, therefore, the center of the gravity of the entire device is lowered, and the mechanical stability is improved.

Furthermore, the rotation generates the rotating magnetic field.

In addition, since the number of electromagnets is reduced, the device is reduced in size.

The endoscope 123 comprises: an elongated insertion unit 131 that is easily inserted in the body cavity; an operating unit 132 that is arranged at the rear end of the insertion unit 131; and a universal cable 127 extended from the operating unit 132. A connector at the back end of the universal cable 127 is connected to a video processor 133 arranged to the control unit 128.

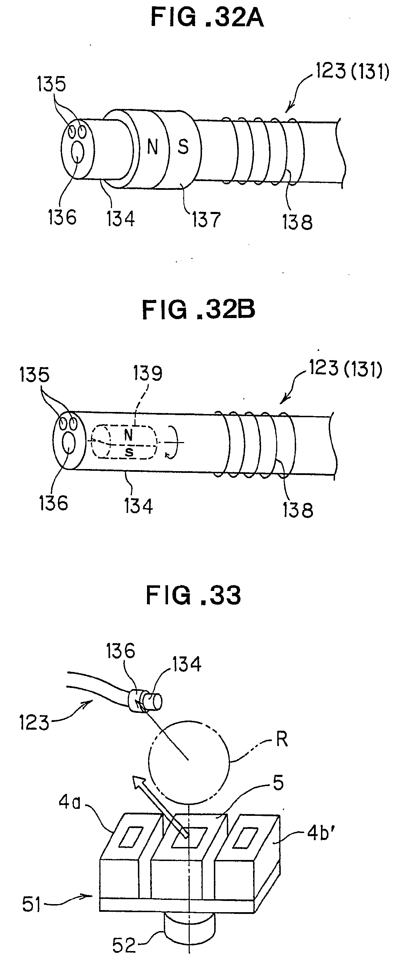

Referring to Fig. 32A, the endoscope 123 comprises: an illuminating window 135 that emits illuminating light and illuminate a subject such as the affected part in the body of a patient; and an observing window 136 that picks-up the image of the illuminated subjected, at a distal-end portion 134 of the insertion unit 131.

According to the second arrangement, the distal-end portion 134 comprises, on the outer-circumference thereof, a magnet 137 that is magnetized in the axial direction of the distal-end portion 134. By using the magnetic field generated by the magnetic field generating unit 51, the magnetic force operates to the magnet 137, thereby changing the direction of the distal-end portion 134.

Further, a coil 138 for generating an alternating magnetic field is arranged near the outer circumference of the distal-end portion 134. The alternating current is flowed to the coil 138, thereby generating the alternating magnetic field. A magnetic position detecting mechanism detects the position and the direction of the coil 138 by detecting the alternating magnetic field by the magnetic sensor 126 shown in Fig. 30.

The magnetic sensor 126 comprises a plurality of magnetic sensor devices, and detects the position of the coil 138 and the axial direction of the coil 38, that is, the direction (posture) of the distal-end portion 134 of the endoscope 123 in the axial direction. The magnetic sensor 126 detects the magnetic field generated by the two-dimensional magnetic field generating unit 51 as well as the alternating magnetic field generated by the coil 138. The two magnetic field signals are separated by filter processing because they have different frequencies.

The signal based on the magnetic field generated by the two-dimensional magnetic field generating unit 51, detected by the magnetic sensor 126, is inputted to the magnetic field control unit in the control unit 128. The magnetic field control unit detects the magnetic field that is actually generated at the position of the distal-end portion 134, and controls the current flowing to the electromagnet of the magnetic field generating unit 51 by the detected information, thereby always generating the optimum magnetic field for magnetically guiding the distal-end portion 134 therenear. Thus, the magnetic field is precisely generated.

The alternating magnetic field signal generated by the coil 138, detected by the magnetic sensor 126, is used to detect the moving speed of the distal-end portion 134 and to detect the acceleration. The information is sent to the control unit 128, thereby realizing the high-level control operation.

Referring to Fig. 32A, the distal-end portion 134 has the circular detachable magnet 137. In this case, the magnet is attached to the existing endoscope, and the conventional endoscope is used. Further, the endoscope has the thin diameter.

According to one modification, referring to Fig. 32B, a magnet 139 may be arranged in the distal-end portion 134, and the magnet 139 may be rotatably accommodated around the central axis of the distal-end portion 134.

The magnet 139 shown in Fig. 32B is magnetized to the N and S poles in the diameter direction. The video processor 133 shown in Fig. 30 comprises a light source device (not shown) for supplying illuminating light. The video processor 133 further comprises a signal processing device that processes the signal of the image pickup signal picked-up by a solid-state image pickup device arranged at the image forming position of an objective optical system of the observing window 136. A video signal generated by the signal processing device is sent to a display unit 238, thereby displaying the image picked-up by the solid-state image pickup device of the endoscope 123 on the display surface of the display unit 238.

The robot arm 124 arranged to the side portion of the bed 31 has a vertical moving mechanism 142 at a main body portion 141, and is movable in the vertical direction on the top end side thereof as shown by an arrow.

Further, a top end 141a of the main body portion 141 is rotatable around the axial direction of the main body portion 141. A rotating member forming the planar moving mechanism 144 is rotatably held at the end of the first arm 143 extended in the horizontal direction from the portion.

At the end portion of a second arm 145 extended in the horizontal direction from the rotating member, a rotating member forming a rotating mechanism 146 is rotatably held. The magnetic field generating unit 51 is attached to the rotating member.

An instruction/operation unit 147 is arranged to the top surface of the control unit 128. The operation of the robot arm 124 is controlled and the direction and the level of the static magnetic field generated by the magnetic field generating unit 51 are changed by operating the instruction/operation unit 147. In another expression, the positional relationship (relative position) between the position of the distal-end portion 134 in the endoscope 123 and the position of the magnetic field generating unit 51 is obtained based on the position of the distal-end portion 134 of the endoscope 123, detected by a magnetic position-detecting mechanism and control information sent to the vertical moving mechanism 142 and the planar moving mechanism 144. The magnetic position-detecting mechanism and the control unit 128 form a relative-position detecting mechanism for obtaining the relative position between the distal-end portion 134 of the endoscope 123 and the magnetic field generating unit 51. The control unit 128 controls the position of the magnetic field generating unit 51 via the vertical moving mechanism 142 and the planar moving mechanism 144 based on the information on the relative position obtained by the relative-position detecting mechanism.

Fig. 33 shows the state of a guiding method for keeping the distal-end portion 134 of the endoscope 123 within the guidable region R by controlling the position of the magnetic field generating unit 51.

Referring to Fig. 33, when the distal-end portion 134 of the endoscope 123 is deviated from the guidable region R of the magnetic field generating unit 51, the position of the distal-end portion 134 is calculated by the output from the magnetic sensor 126, and the vertical moving mechanism 142 and the planar moving mechanism 144 are controlled based on the positional information via the control unit 128, and the distal-end portion 134 of the endoscope 123 is caused to exist within the guidable region R of the magnetic field generating unit 51.

In the case shown in Fig. 33, the magnetic field generating unit 51 is moved in a direction shown by a thick arrow including a white portion and thus the distal-end portion 134 of the endoscope 123 is caused to exist within the guidable region R.

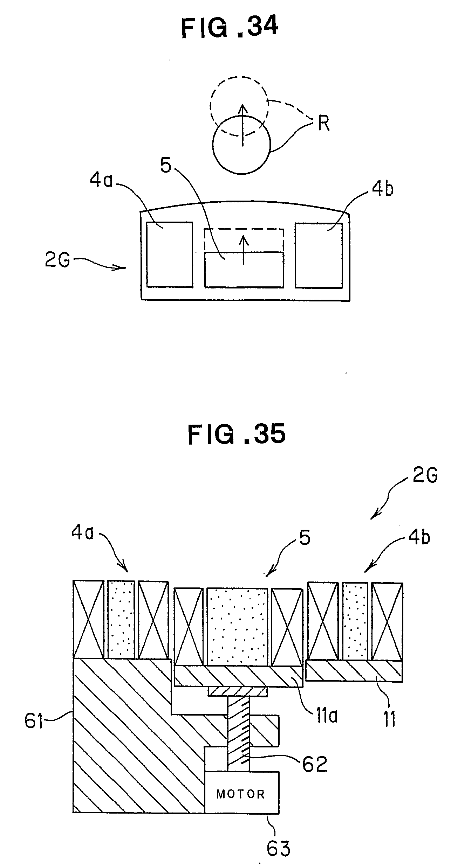

In place of moving the component in the vertical direction of the magnetic field generating unit, as shown by a magnetic field generating unit 2G shown in Fig. 34, the guidable region R may be moved in the vertical direction by moving the third electromagnet 5.

The structure of the magnetic field generating unit 2G in this case is shown in Fig. 35.

Fig. 35 shows the magnetic field generating unit 2G for adjusting the position (adjusting the movement) of the third electromagnet 5 arranged in the center in the direction of the central axis O. For example, a bottom portion 11a under the central electromagnet 5 in the base 11 is fit into a hole, and is movable in the up/down direction.

The bottom portion 11a is held by the distal end of a screw 62 screwed into a screw hole of a holding unit 61 connected to a bottom portion on the outer circumference. A motor 63 is arranged at the bottom end of the screw 62. The motor 63 is rotated forward or backward based on the positional information, thereby elevating the bottom portion 11a and varying the height position of the central electromagnet 5 to adjust the generated magnetic field.

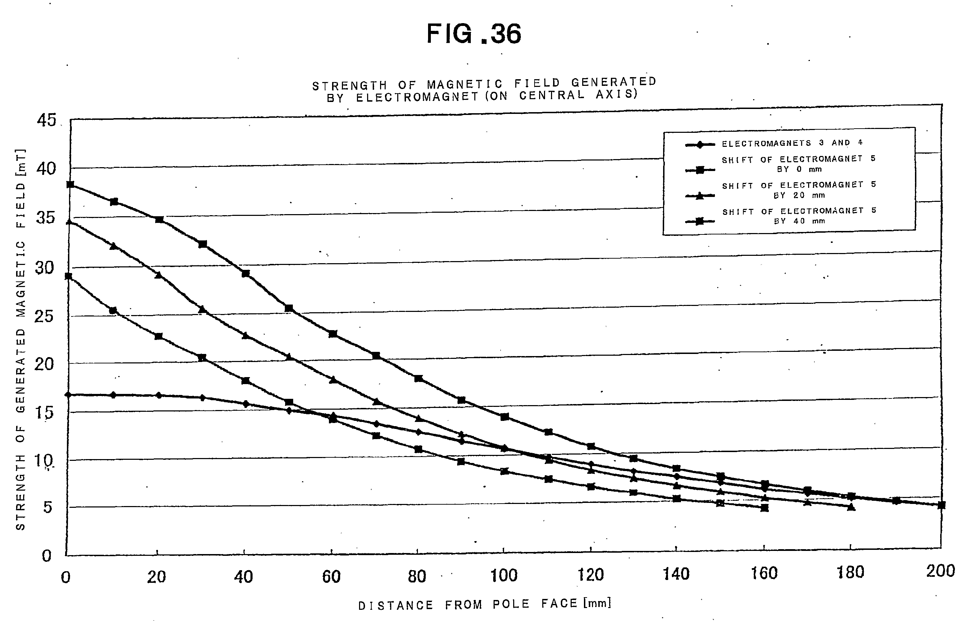

Fig. 36 shows a measurement result of the generated magnetic field in the case of changing the height of the third electromagnet 5 arranged in the center. By changing the height of the third electromagnet 5, the values of the generated magnetic field is adjusted in accordance with the magnetic fields generated by the peripheral electromagnets 3a and 3b.

That is, since the characteristics of the generated magnetic field relative to the distance from the pole face are slightly different between the central electromagnet 5 and the peripheral electromagnets 3a and 3b, the magnetic field in the desired direction and with the desired strength is easily generated relative to the target distance by adjusting the height of the central electromagnet 5 in accordance with the values of the generated magnetic field relative to the distance from the pole face. In this case, the same advantages as those in the case of controlling the current of the electromagnets depending on the distance D according to the first embodiment are obtained.

Incidentally, according to the first embodiment, the same advantages are obtained by changing the height of the electromagnet 5 relative to the electromagnets 3a and 3b and the electromagnets 4a and 4b. Further, according to the second arrangement, similarly to the first embodiment, the current of the electromagnets may be controlled by the distance D.

As mentioned above, according to the second arrangement, the static magnetic force is applied to the magnet 137 or 139 by applying the static magnetic field to the magnet 137 or 139 arranged to the endoscope 123, thereby changing the direction of the endoscope 123 in the desired direction.

Therefore, the user controls the direction of the static magnetic field, thereby changing the distal-end portion 134 of the endoscope 123 in the desired direction. Further, the insertion into the body cavity is easy and the observing direction is changed in the desired direction.

### (Third embodiment)

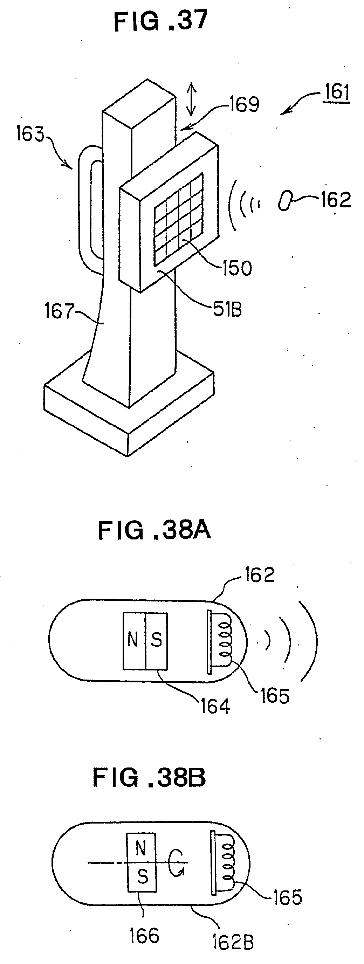

Next, a description is given of a third embodiment of the present invention with reference to Fig. 37. Fig. 37 shows a magnetic guiding medical system 161 according to the third embodiment of the present invention. According to the third embodiment, similarly to the second embodiment, a magnetic field generating unit 51B for guiding operation, arranged outside the body, applies the magnetic field to a magnet 164 or 166 (refer to Figs. 38A and 38B) in a capsule medical apparatus 162, thereby controlling the direction of the capsule medical apparatus 162 with the magnetic force operating to the magnet 164 (or 166) in the capsule medical apparatus 162.

The magnetic guiding medical system 161 comprises: a capsule medical apparatus 162 that is inserted in the body and picks-up images inside the body; and an extracorporeal device 163 that receives image information sent by wireless manner from the capsule medical apparatus 162.

Referring to Fig. 38A, the capsule medical apparatus 162 comprises the magnet 164 and a sending antenna 165. Similarly to the capsule medical apparatus 72 shown in Fig. 3, the capsule medical apparatus 162 further comprises illuminating means and image pickup means. Although the magnetizing direction of the magnet 164 corresponds to the axial direction of the capsule medical apparatus 162 as shown in Fig. 38A, the magnet 166 magnetized in the diameter direction in the rotatable state around the central axis of the capsule medical apparatus 162 may be used as shown in Fig. 38B.

The extracorporeal device 163 has a control unit (not shown) in a main body 167 which is substantially quadratic-prism-shaped and stands in the up/down direction. A planar moving mechanism 169 is arranged in front of the quadratic prism, and the planar moving mechanism 169 holds the magnetic field generating unit 51B and a receiving antenna unit 150 in front thereof that are movable in the up/down direction. In this case, the magnetic field generating unit 51B and the receiving antenna unit 150 are moved in the up/down direction, that is, one-axial direction. The planar state of the magnetic field generating unit 51B and the receiving antenna unit 150 is held and, simultaneously, they are slidable in the up/down direction. The receiving antenna unit 150 has the similar function of the receiving antenna unit 73 according to the first embodiment. Similarly to the first embodiment, the receiving antenna unit 150 has a function for detecting the position of the capsule medical apparatus 162. The receiving antenna unit 150 is held by the magnetic field generating unit 51B. Therefore, the detected position of the capsule medical apparatus 162 obtained by using the receiving antenna unit 150 indicates the position and posture relationship (relative position/posture) between the capsule medical apparatus 162 and the magnetic field generating unit 51B. The position/posture of the magnetic field generating unit 51B is controlled by the detected position/posture relationship.

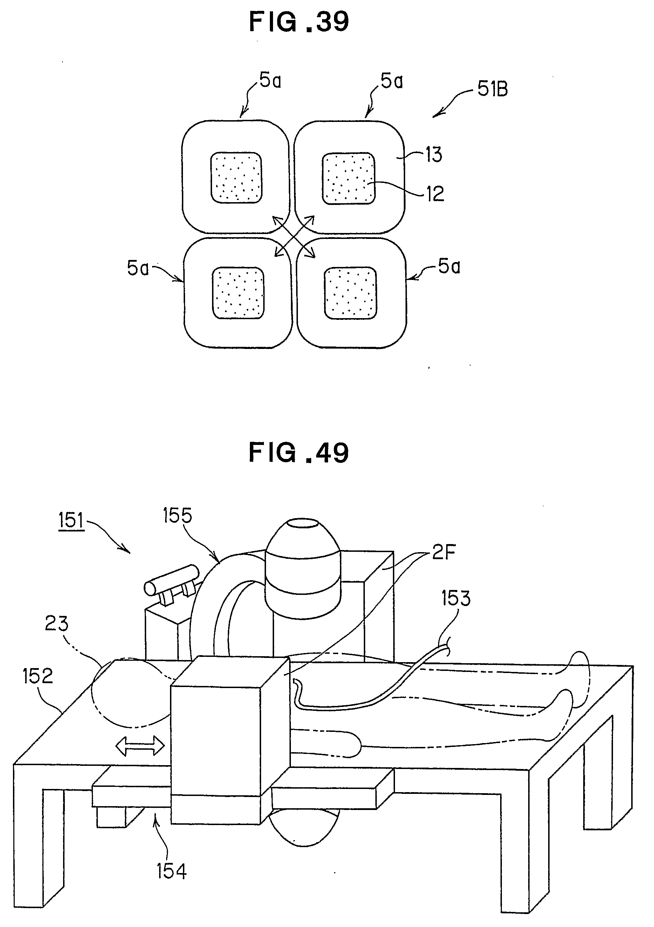

Fig. 39 shows the structure of the magnetic field generating unit 51B according to the third embodiment.

The magnetic field generating unit 51B shown in Fig. 39 is a device for generating the two-dimensional magnetic force. In the two-dimensional magnetic field generating unit 51B, the electromagnets having the same characteristics, e.g., two of four electromagnets 5 are individually arranged adjacently in the up/down (longitudinal) and horizontal (lateral) direction, thereby generating the two-dimensional magnetic field as shown by an arrow in Fig. 39.

The magnetic field generating unit 51B has two sets of electromagnets with high symmetricalness, as compared with the magnetic field generating unit 51 shown in Fig. 31A. Therefore, the magnetic field in the more uniformizing direction is generated at the central axis.

According to the third embodiment, it is possible to smoothly guide the direction of the capsule medical apparatus 162 for medical action, such as examination using the endoscope, which is inserted into the body cavity, with the magnetic field.

Next, a description is given of magnetic field generating unit according to modifications. The modifications are obtained by modifying or improving the magnetic field generating unit 2 according to the first embodiment. First, a description is given of the case of increasing the generated magnetic field by efficiently using the space.

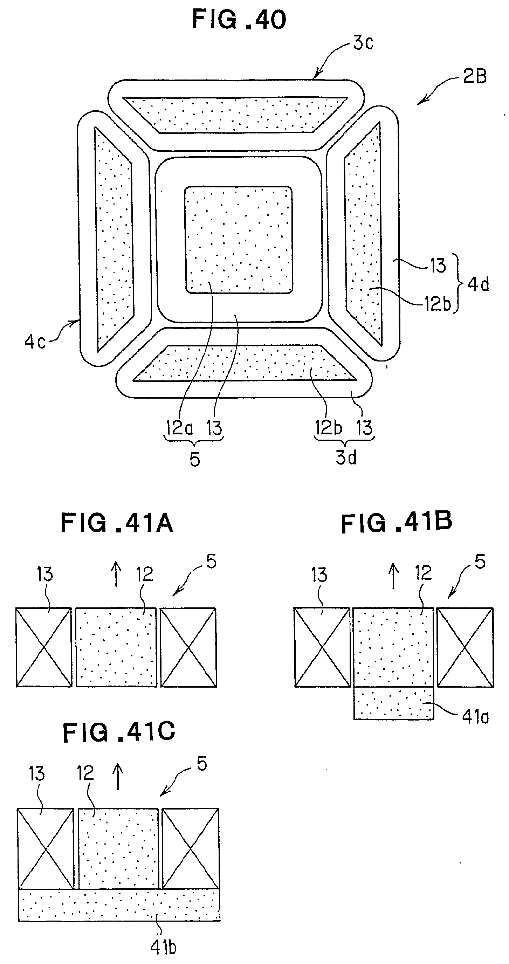

Fig. 40 shows a magnetic field generating unit 2B according to a first modification. The magnetic field generating unit 2B uses electromagnets 3c and 3d and the electromagnets 4c and 4d which are obtained by trapezially shaping the electromagnets 3a and 3b and the electromagnets 4a and 4b in the magnetic field generating unit 2 shown in Fig. 9.

The electromagnet 5 in the center is formed by winding a coil 13 to a ferromagnetic member unit 12a containing a column-shaped ferromagnetic member having a quadrate cross-section with high permeability. Incidentally, the region R is formed by removing four corners of the ferromagnetic member unit 12a.

In this case, the radius of the region R is a minimum bend radius of the wiring forming the coil 13 and then the wiring has high density.

The electromagnets 3c and 3d and the electromagnets 4c and 4d, which are isosceles-trapezoid-shaped, are arranged around the electromagnet 5 in the center to be close to the outer surface of the coil 13 which is substantially planar. That is, the electromagnets 3c and 3d and the electromagnets 4c and 4d are symmetrically arranged so that the short side corresponds to the inside. The inclined portions of the electromagnets 3c and 3d and the electromagnets 4c and 4d are arranged to be close to the adjacent inclined portions (in the electromagnets 3c and 3d and the electromagnets 4c and 4d) in parallel with each other.

Ferromagnetic member units (magnet core portions) 12b forming the electromagnets 3c and 3d and the electromagnets 4c and 4d comprise isosceles-trapezoid-shaped column members containing ferromagnetic members. The coil 13 is wound to the ferromagnetic member units 12b, thereby forming the electromagnets.

As mentioned above, the electromagnets 3c, 3d, 4c, 4d, and 5 are in close formation on the plane, and the region of close formation hardly has any spaces not shared by the electromagnet. A high magnetic field is efficiently generated.

Next, a description is given of the case of strengthening the generated magnetic field by adding a ferromagnetic member. Fig. 41A shows the cross section of the electromagnet 5 according to the first embodiment. For example, referring to Fig. 41B, a ferromagnetic member 41a is arranged at one end of the electromagnet 5 (on the opposite side of the generation of magnetic field). Or, the arranged ferromagnetic member 41a matches the outer shape of the electromagnet 5.

Referring to Fig. 41C, the outer shape of a ferromagnetic member 41b substantially matches the outer shape of the electromagnet 5 on the bottom side thereof.

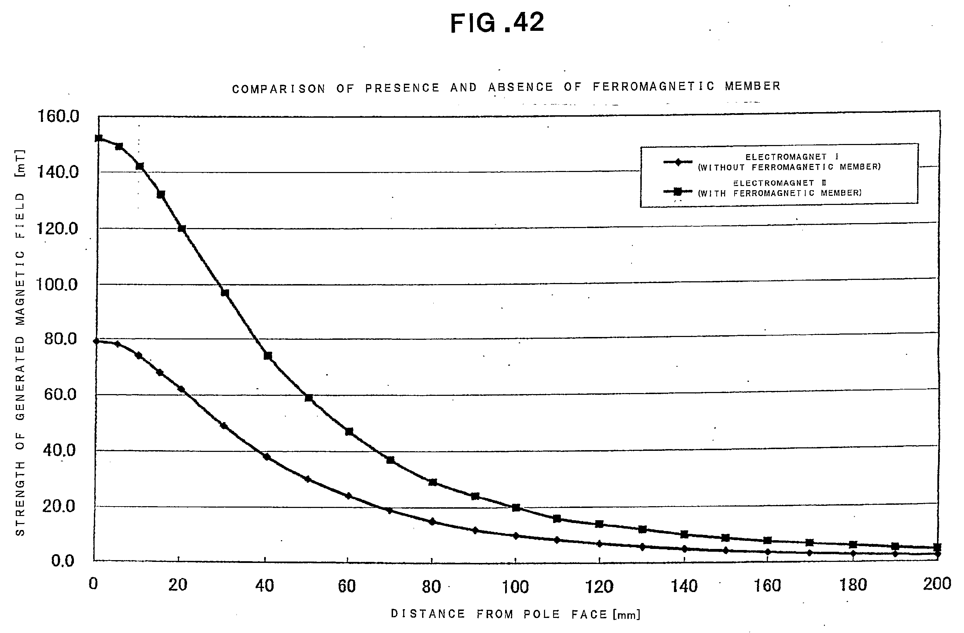

Fig. 42 shows the generated magnetic field without the arrangement of the ferromagnetic member as shown in Fig. 41A (according to the first embodiment) and the generated magnetic field with the arrangement of the ferromagnetic member 41b. As will be understood with reference to Fig. 42, the generated magnetic field is more increased with the ferromagnetic member 41b by twice than the case without the ferromagnetic member 41b.

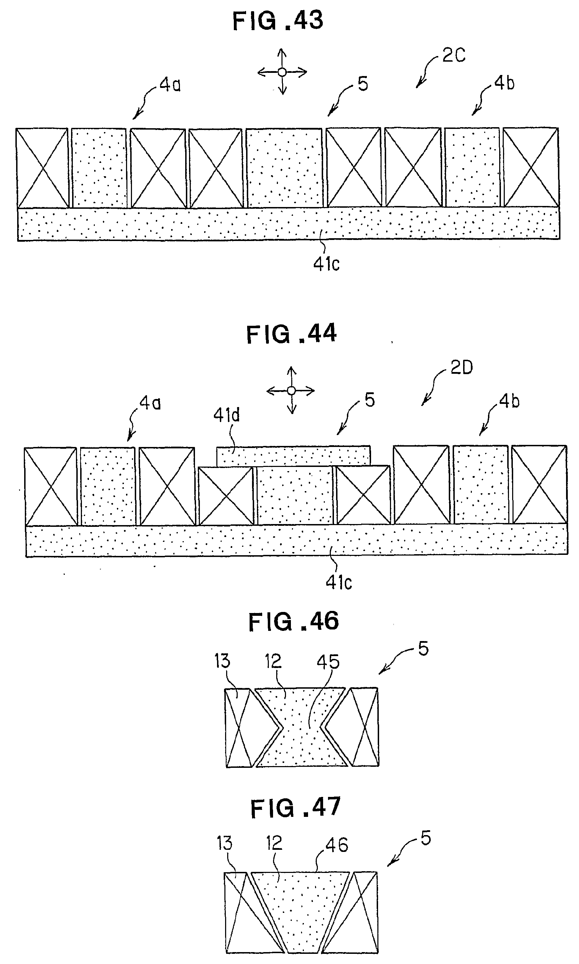

Incidentally, in addition to the cases with reference to Figs. 41B and 41C, referring to Fig. 43, a magnetic field generating unit 2C may be structured by arranging a plurality of electromagnets 5, 4a, 4b, etc. onto a plate of a ferromagnetic member 41c having the outer shape of the entire electromagnets. Although not shown in Fig. 43, a plate of the ferromagnetic member 41c is arranged under the electromagnets 3a and 3b. Fig. 43 shows the case according to the first embodiment. With reference to Fig. 40, according to the first modification, the electromagnets 4a and 4b correspond to the electromagnets 4c and 4d.

Incidentally, the ferromagnetic members 41a may be provided independently of the magnet core portion 12 containing the ferromagnetic member. However, the ferromagnetic members 41a which are provided integrally with the magnet core portion 12 easily suppresses the leakage magnetic flux. Thus, the generated magnetic field is further increased.

Next, a description is given of the advantageous structure for uniformizing the magnetic field.

The cross-sectional area of the magnet core portion containing the ferromagnetic member of the electromagnet 5 in the center is wider than those of the ferromagnetic members of the electromagnets 3a and 3b (or 3c and 3d) and the electromagnets 4a and 4b (or 4c and 4d) which are peripherally arranged. This arrangement is used according to the first embodiment.

In addition, on the generation side of the magnetic field of the electromagnet 5 in the center, a ferromagnetic member 41d having the cross-sectional area larger than that of the magnet core portion of the electromagnet is arranged. Fig. 44 shows a magnetic field generating unit 2D in this case. Incidentally, the magnetic field generating unit 2D is applied to the case of the magnetic field generating unit 2C shown in Fig. 43.

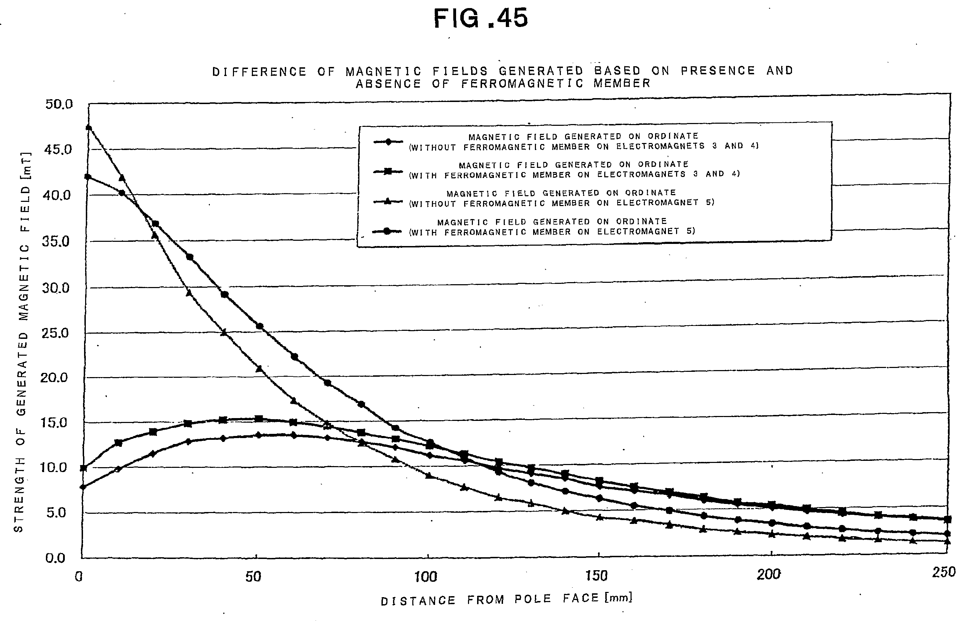

Fig. 45 shows measurement results (without the indicating the efficiency with the arrangement of the ferromagnetic member 41c on the bottom side) in the case of arranging a ferromagnetic member 41d onto the top of the electromagnet 5 in the center as shown in Fig. 44. Referring to Fig. 45, with the arrangement of the ferromagnetic member 41d, the large change of the strength of magnetic field near the magnetic pole is suppressed and the strength of magnetic field is increased at the far distance from the near portion, as compared with the case without the arrangement of the ferromagnetic member 41d.

In addition, the central portion of the core containing the ferromagnetic member of the electromagnet 5 in the center is caved. Fig. 46 shows the electromagnet 5 in the center in this case. The electromagnet 5 includes a caved portion 45 having the narrowest cross-sectional area in the center in the height direction. As closer to an end portion of the portion in the height direction, the cross-sectional area increases.

In addition, the ferromagnetic member of the electromagnet 5 in the center is widened toward the side for generating the magnetic field. Fig. 47 shows the electromagnet 5 in the center in this case. In the electromagnet 5, the magnetic field is generated on the top side of the sheet and, therefore, a maximum area portion 46 having the widest cross-sectional area on the top end of the magnet core portion 12 is formed.

With the structure shown in Fig. 44, advantageously, the generated magnetic field is uniformized. Further, a magnetic field generating unit 2E may be used as shown in Fig. 48.

In the magnetic field generating unit 2E, a ferromagnetic member 41e is arranged onto the pole face on the generating side of the magnetic field of the peripheral electromagnets (although the electromagnets 4a and 4b are shown, the foregoing is applied to the electromagnet 3a and 3b).

Fig. 45 shows measurement results of the influence with the arrangement of the ferromagnetic member 41e and without the arrangement of the ferromagnetic member 41e.

As will be understood with reference to Fig. 45, with the ferromagnetic member 41e, the strength of magnetic field is increased near the pole face, and the uniformized magnetic field is generated at the distance sufficiently far from the pole face.

### (Fourth embodiment)

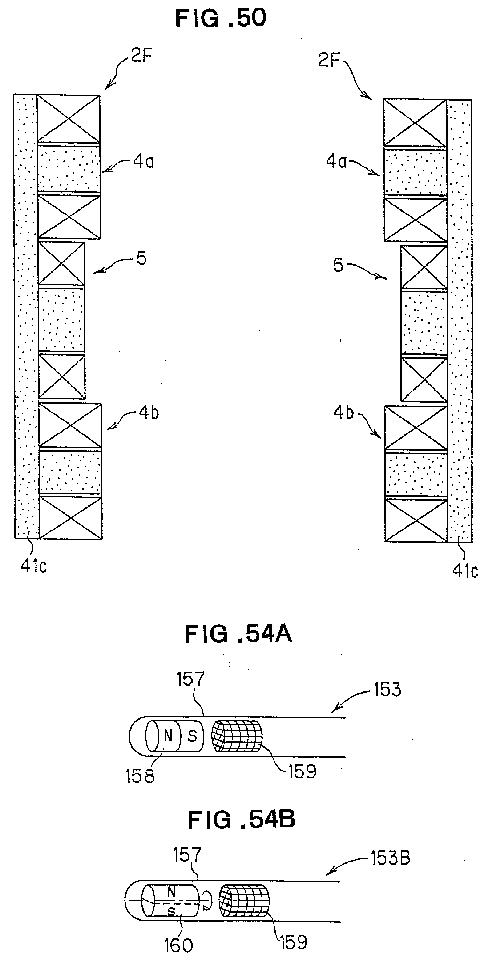

Next, a description is given of a fourth arrangement not according to the present invention with reference to Fig. 49. The fourth arrangement basically uses the second arrangement, and only the features according to the fourth arrangement will be described. Fig. 49 shows a magnetic guiding medical system 151 according to the fourth arrangement of the present invention. According to the fourth arrangement, the magnetic field generating units 2F for guiding operation, which are extracorporeally arranged to face each other, apply the static magnetic field to a magnet 158 (or, refer to a magnet 160 in Figs. 54A and 54B) in a catheter 153, the direction of the catheter 153 is controlled by the magnetic force operating to the magnet 158 (or 160) in the catheter 153.

The magnetic guiding medical system 151 comprises: the catheter 153 which is inserted in the body of the patient 23 laid on a bed 152; the magnetic field generating units 2F which are arranged to face the side of the bed 152; a planar moving mechanism 154 which is arranged on the bed 152 to move the magnetic field generating units 2F in parallel with each other; a fluoroscopic device 155, such as an X-ray fluoroscopic device for the body of the patient 23; and a control unit (not shown).

The patient 23 is viewed through the fluoroscopic device 155, and the position of the distal end of the catheter 153 is detected. The position information is used for controlling the direction of the distal end of the catheter 153 in the desired direction. That is, the fluoroscopic devices 155 functions as a fluoroscopic-type position detecting mechanism which detects the position/posture of the distal end of the catheter 153. Further, a position/posture relationship (relative position/posture) between the distal end of the catheter 153 and the magnetic field generating units 2F is obtained based on an output from the fluoroscopic-type position detecting mechanism and the control information for controlling the planar moving mechanism 154 which changes the position of the magnetic field generating units 2F, from the control unit (128 according to the second arrangement). That is, the fluoroscopic device 155 and the control unit form the relative position/posture detecting mechanism. Based on the relative position/posture information, the position/posture of the magnetic field generating unit 2F is controlled by the control unit.

Fig. 50 shows the structures of the magnetic field generating units 2F.

Referring to Fig. 50, the magnetic field generating units 2F may be arranged, facing each other, thereby generating a desired three-dimensional magnetic field in the center. Although this case requires the space for facing arrangement of the magnetic field generating units 2F on both sides of the central position, the arrangement is excessively advantageous when the space is available.

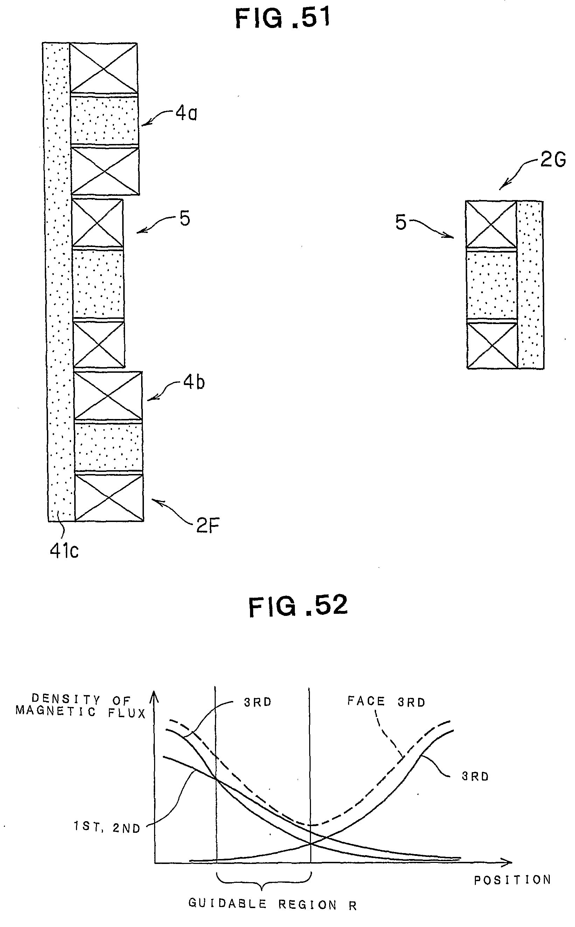

Incidentally, referring to Fig. 50, in place of the facing arrangement of the two magnetic field generating units 2F, one of the magnetic field generating units 2F may be the third electromagnet 5. Fig. 51 shows the magnetic field generating unit in this case.

Fig. 52 shows the characteristics of the generated magnetic field in the case shown in Fig. 51.

A solid line shows the density of magnetic flux to the positions of the first, second, and third electromagnet units. On the other hand, a dotted line shows the facing arrangement of the third electromagnet unit 5 to one part. As will be understood from the characteristics shown by the dotted line, at the far position from the magnetic field generating unit, the magnetic field is increased and the smooth magnetic field is generated (with the small change in strength of magnetic field and in angle of magnetic field, relative to the space).

Referring to Fig. 52, the third electromagnet unit 5 has a lower magnetic field at the far position from the third electromagnet unit 5, as compared with other electromagnets. Therefore, the compensation of the reduction in magnetic field in the region by using the facing electromagnet generates the smooth magnetic field and widens the guidable region.

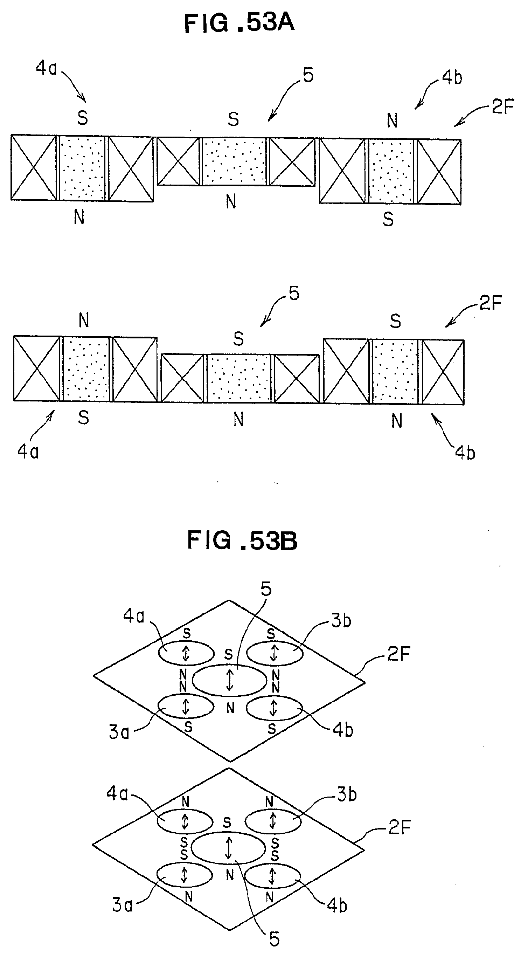

Incidentally, Figs. 53A and 53B show the magnetizing directions of the facing electromagnets. Fig. 53A shows the magnetizing direction with the cross section, and Fig. 53B schematically shows the magnetizing direction of facing the electromagnets in the up and down directions. Figs. 53A and 53B show the case shown in Fig. 50, including the case shown in Fig. 51.

Further, Fig. 54A shows the structure of the catheter 153 on the distal-end side. A distal end 157 of the catheter 153 comprises a magnet 158 magnetized in the axial direction and a magnetic sensor 159.

The magnetic sensor 159 controls the magnetic field with higher precision. Since the direction of the distal end of the catheter is limited to some degree by the lumen in the body, the direction of the generated magnetic field does not necessarily match the direction of the catheter 153. Then, the magnetic field which is actually generated at the distal end of the catheter is calculated with high precision based on an output value from the magnetic sensor 159 and the position/posture obtained by the fluoroscopic device 155. Thereby, the magnetic field is generated with high precision and stability by the feedback operation of the difference between the direction of the magnetic field to be actually generated and the magnetic field that is actually generated.

Incidentally, the magnetic sensor 159 detects the strength and the direction of the generated magnetic field, that is, the static magnetic field, generated by the magnetic field generating units 2F for guiding operation. The detected signal, by connecting a signal line (not shown) extended from the rear end of the catheter 153 to the control unit, is inputted to a position detecting unit of the control unit. The calculated position and direction are displayed on a display unit (not shown), thereby enabling the replacement of the fluoroscopic device 155.

The user refers to information displayed on the display unit and operates the instruction/operation unit, thereby controlling the direction of the distal end part of the catheter 153.

Incidentally, referring to Fig. 54B, the magnet 160 magnetized in the diameter direction may be rotatably accommodated in a catheter 153B in the axial direction thereof.

### (Fifth arrangement)

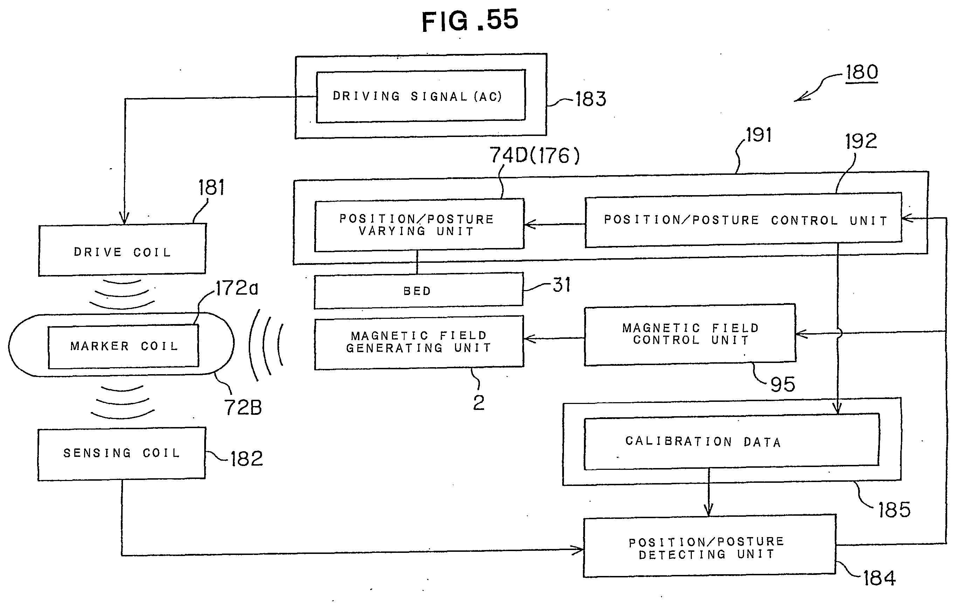

Next, a description is given of the fifth arrangement with reference to Figs. 55 to 64B. The fifth arrangement corresponds to modifications of the first and second arrangements. The features according to the fifth arrangement will be described. Fig. 55 shows the structure of a magnetic guiding medical system 180 according to the fifth arrangement .

Although the position/posture varying unit mainly moves the magnetic field generating unit 2, thereby changing the position/posture according to the first embodiment, the magnetic field generating unit 2 is fixed and a position/posture varying unit 74D of the bed 31 varies the position/posture in the magnetic guiding medical system 180 according to the fifth arrangement, thereby magnetically guiding the system. The position/posture varying unit 74D is controlled by a position/posture control unit 192 forming a control unit 191. The magnetic field generating unit 2 is controlled by a magnetic field control unit 95.

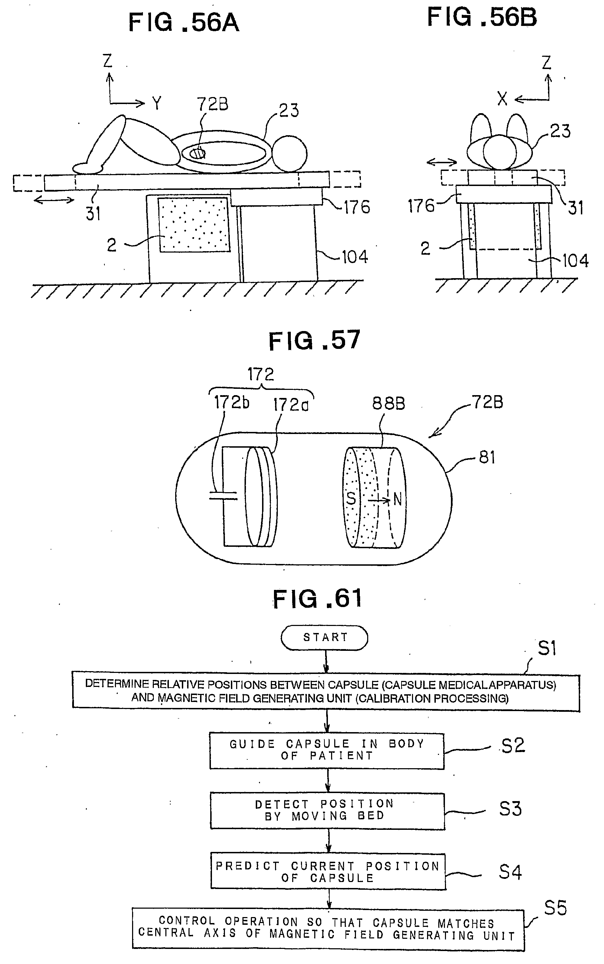

Specifically, referring to Figs. 56A and 56B, the position/posture varying unit 74D comprises a bed horizontal moving mechanism 176 for moving the bed 31 on which the patient 23 is placed on the horizontal plane.

Referring to Figs. 56A and 56B, the bed horizontal moving mechanism 176 arranged onto the top surface of the bed supporting base 104 moves the bed 31, serving as a target placing unit, on which the patient 23 is placed, in the Y direction and the X direction. Under the bed 31, the magnetic field generating unit 2 is fixed. According to the fifth arrangement, although the target placing unit comprises the bed 31, it may be chair-shaped, bath-shaped, or toilet-bowl-shaped.

Further, according to the fifth arrangement, referring to Fig. 55, a capsule medical apparatus 72B includes a marker coil 172a. A drive coil 181 and a sensing coil 182 detect the position/posture of the marker coil 172a.

The drive coil 181 is driven by a drive-signal generating unit 183, and the signal detected by the sensing coil 182 is inputted to a position/posture detecting unit 184.

The signal detected by the position/posture detecting unit 184 is outputted to the position/posture control unit 192 of the control unit 191 and the magnetic field control unit 95, thereby being used for control operation thereof.

The calibration data is used so as to precisely detect the position/posture of the marker coil 172a. Therefore, according to the fifth arrangement, a calibration data storing unit 185 for storing the calibration data is arranged, and the calibration data is used for the detection of the position/posture using the position/posture detecting unit 184.

Fig. 57 shows a capsule medical apparatus 72B. The capsule medical apparatus 72B comprises a magnet 88B which is arranged in the capsule container 81 so that the magnetizing direction of the magnet 88B matches the axial direction of the container 81. The direction of the capsule medical apparatus 72B is controlled to be in the direction of the magnetic field.

As mentioned above, the container 81 comprises the marker coil 172a for detecting the position of the capsule medical apparatus 72B. The marker coil 172a and a condenser 172b form a resonant circuit 172 which is resonant at a predetermined frequency. Incidentally, the container 81 accommodates therein the image pickup device 84 shown in Fig. 3 and the like (not shown).

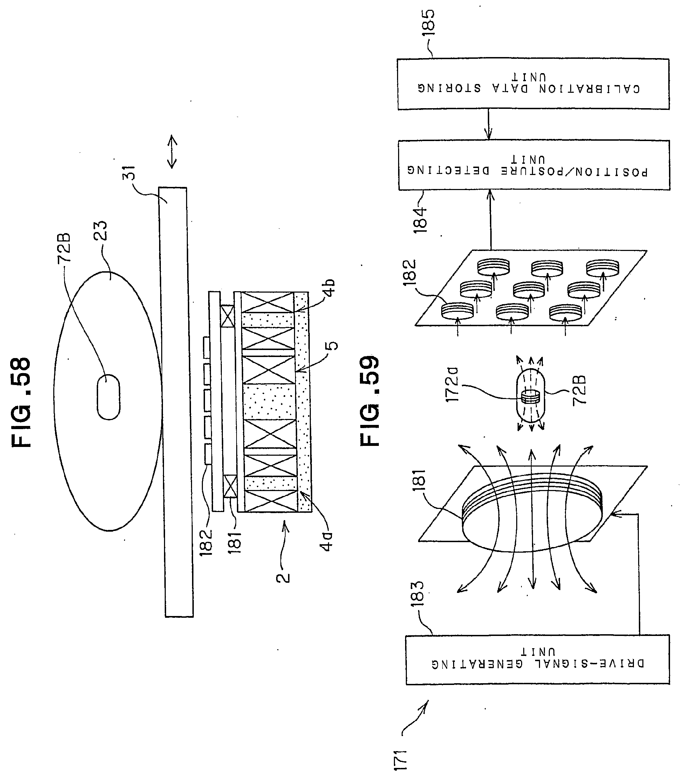

Fig. 58 shows an arrangement example of the magnetic field generating unit 2 and the drive coil 181 and the sensing coil 182, serving as driving and detecting means, for detecting the position of the capsule medical apparatus 72B.

Under the bed 31, the magnetic field generating unit 2 is arranged. The magnetic field generating unit 2 comprises five electromagnets arranged on the plane, and has the structure shown in Fig. 9.

Further, under the bed 31, the drive coil 181 for generating the alternating magnetic field is fixed onto the top surface of the magnetic field generating unit 2.

The above-mentioned arrangement of the drive coil 181 always keeps a relative positional relationship between the marker coil 172a and the drive coil 181 to be under a stable and high detecting-precision condition, under which the marker coil 172a exists within a strong magnetic field generated by the drive coil 181. This is because the control operation is performed to prevent the large change in relative position between the electromagnets in the magnetic field generating unit 2 and the capsule medical apparatus 72B.

Referring to Fig. 58, a plurality of sensing coils 182 are fixed onto the top surface of the magnetic field generating unit 2, specifically, top surface of the drive coil 181. The above-mentioned arrangement of the sensing coil 182 controls the electromagnets of the magnetic field generating unit 2, which trace the capsule medical apparatus 72B. Therefore, the relative position between the marker coil 172a and the sensing coil 182 is controlled under a stable and high-precision condition.

The ferromagnetic member of the magnetic field generating unit 2 influences on the coil characteristics of the drive coil 181 and the sensing coil 182. However, the above-mentioned structure prevents the change in positional relationship between the magnetic field generating unit 2 and the sensing coil 182 and drive coil 181 if the position of the magnetic field generating unit 2 changes. Therefore, the characteristics of the sensing coil 182 and the drive coil 181 do not change, and the detecting precision is improved.

Under the control operation, under which the relative positional relationship between the electromagnet of the magnetic field generating unit 2 and the capsule medical apparatus 72B does not change, the relative positional relationship between the marker coil 172a and the drive coil 181 and sensing coil 182 does not change. Therefore, with the sensing coil 182, serving as the reference, the control operation does not have any problems in the narrow detected region for detecting the position or posture of the capsule medical apparatus 72B by the position/posture detecting unit 184. Thus, the number of sensing coils 182 is reduced, the amount of calculation for obtaining the position/posture is reduced, and the algorithm for obtaining the position/posture is simple.

In particular, when the drive coil 181 is fixed to the magnetic field generating unit 2, the magnetic field generating unit 2, which influences on the magnetic field for detecting the position, is integrated to both the coils (drive coil 181 and sensing coil 182) for detecting the position. Therefore, the change in calibration data due to the change of the planar moving mechanism (position/posture varying unit) is small. The position is stably detected (because the change in output of the drive coil is small relative to the output of the marker coil).

Further, the calibration data is influenced from the change in relative position among the drive coil 181, the sensing coil 182, and the ferromagnetic member in the chamber. According to the fifth arrangement, when the drive coil 181 and the sensing coil 182 are fixed to the base together with the magnetic field generating unit 2, the change of the planar moving mechanism (position/posture varying unit) does not change the relative position among the drive coil 181 and sensing coil 182 and the magnetic field generating unit 2, and the ferromagnetic member in the chamber. Thus, the change of the planar moving mechanism (position/posture varying unit) does not change the influence of the ferromagnetic member in the chamber, and the amount of change in the calibration data due to the change of the planar moving mechanism (position/posture varying unit) is small. As a consequence, the position is stably detected.

Referring to Figs. 55 and 59, the drive coil 181 is connected to the drive-signal generating unit 183 for generating a drive signal, serving as an alternating signal. The drive coil 181 receives the drive signal, thereby generating the alternating magnetic field as shown in Fig. 59. The alternating magnetic field is applied to the capsule medical apparatus 72B. The marker coil 172a in the capsule medical apparatus 72B receives the alternating magnetic field and generates the guiding current. Further, the marker coil 172a generates an alternating magnetic field (guiding magnetic field) generated by the guiding current.

The plurality of sensing coils 182 receive both the alternating magnetic field generated by the drive coil 181 and the alternating magnetic field generated by the marker coil 172a, and output the detected data. Here, the information on the alternating magnetic field generated by the drive coil 181 is processed by processing using calibration data, which will be described later, thereby being canceled. As a result, only the information of the alternating magnetic field generated by the marker coil 172a is obtained by the detected data of the plurality of sensing coils 182.

Referring to Fig. 59, the plurality of sensing coils 182 are connected to the position/posture detecting unit 184, the detected data detected by the plurality of sensing coils 182 is outputted to the position/posture detecting unit 184. The position/posture detecting unit 184 detects (calculates) the position/posture of the capsule medical apparatus 72B based on the inputted detected data.

Fig. 59 shows the structure of a position/posture detecting mechanism 171 for detecting the position and posture of the capsule medical apparatus 72B and the detecting principle according to the fifth arrangement.

The position/posture detecting mechanism 171 comprises: the marker coil 172a arranged in the capsule medical apparatus 72B inserted in the patient 23, serving as the living body; the drive coil 181 and the plurality of sensing coils 182 (or may be magnetic sensors) arranged outside the patient 23; the drive-signal generating unit 183 for generating the alternating magnetic field to the drive coil 181; the position/posture detecting unit 184 for calculating the position or the posture of the capsule medical apparatus 72B based on output signals from the sensing coils 182; and the calibration data storing unit 185 for storing the calibration data.

Here, the calibration means that, before guiding (inserting) the capsule medical apparatus 72B including the marker coil 172a into the patient 23, that is, in the state in which the marker coil 172a is not arranged in the detected area, only the drive coil 181 is driven, the alternating magnetic field is generated, and the strength of magnetic field is then measured. The calibration data indicates the data on the strength of magnetic field measured in this case.

The drive coil 181 generates the alternating magnetic field by supplying a drive signal from the drive-signal generating unit 183. Guiding current flows to the marker coil 172a by using the alternating magnetic field, and the alternating magnetic field is additionally generated. The plurality of sensing coils 182 are arranged and detect the strength of magnetic field generated by the drive coil 181 and the sensing coils 182 at the arrangement positions thereof.

The position/posture detecting unit 184 detects the position or the posture of the marker coil 172a by dipole approximation, or the like, of the magnetic field of the marker coil 172a based on the difference between the outputs of the sensing coils 182 and the data (calibration data) of the strength of magnetic field generated only by the drive coil 181 measured before guiding the capsule medical apparatus 72B into the patient 23.

The following advantages are obtained by using the structure shown in Fig. 59 and the position/posture detecting principle.

That is, the position detection using the magnetic field suppresses the influence from the attenuation due to the living body, thereby detecting the position with high precision. Due to detecting the position by using the alternating magnetic field, the generation of the alternating magnetic field of another frequency by the magnetic field generating unit 2 does not influence on the positional detection with the arrangement of a filter for limiting a frequency band to the sensing coils 182.

Incidentally, the magnetic field generated by the drive coil 181 may be a pulse magnetic field. The generated pulse magnetic field guides the current to the marker coil 172a, and the alternating magnetic field is generated while the resonant circuit 172 attenuates the current. The sensing coil 182 detects the magnetic field in this case. In this case, since only the magnetic field of the marker coil 172a is detected by the sensing coil 182, the calibration is not necessary and the system structure is simplified.

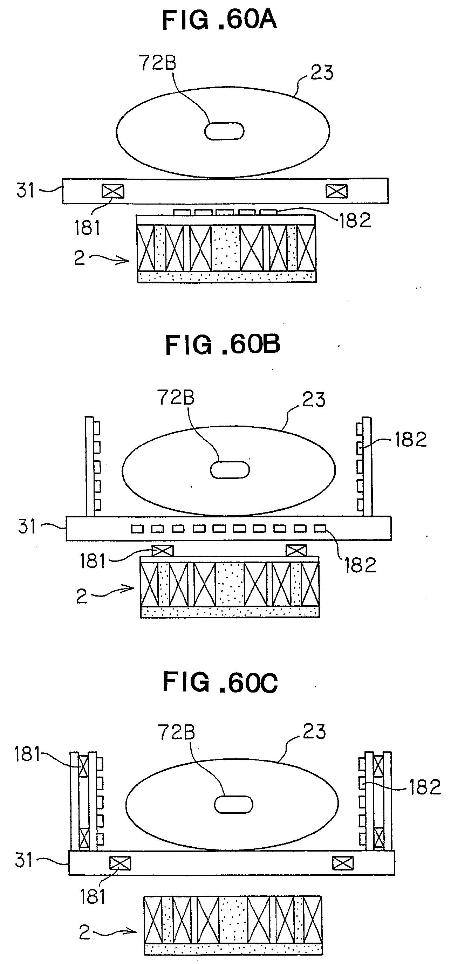

Fig. 60A shows the arrangement positions of the magnetic field generating unit 2, the drive coil 181, and the sensing coils 182 shown in Fig. 58 according to one modification.

Referring to Fig. 60A, the drive coil 181 is fixed to the bed 31. Thus, the size of the drive coil 181 is increased. As the size of the drive coil 181 is increased, a wide magnetic field is efficiently generated.

Further, since the magnetic field generating unit 2 and the drive coil 181 are not moved together therewith, the increase in size of the drive coil 181 does not influence on the size (width and length) of the bed 31 and the size of device is therefore reduced.

Referring to Fig. 60B, the structure shown in Fig. 58 is changed by fixing the sensing coil 182 to the inside of the bed 31 and both sides of the bed 31. According to the one modification, the number of sensing coils 182 is increased.

By arranging the sensing coils 182 to the bed 31, the magnetic field generating unit 2 and the sensing coils 182 are separated. If the sensing coils 182 are widely arranged so as to increase the detecting range, this does not influence on the size and the movable range (width and length) of the bed 31 and the size of device is therefore reduced.

The sensing coil 182 has the coil detecting characteristics that change near the magnetic field generating unit 2, serving as a ferromagnetic member. Referring to Fig. 60B, the sensing coils 182 are arranged to the bed 31, thereby increasing the distance of the sensing coils 182 from the magnetic field generating unit 2. Further, the change in characteristics of the sensing coils 182 due to the change of the position/posture varying unit is suppressed, and the position is detected with higher precision.

Referring to Fig. 60C, in the structure shown in Fig. 60A, further, the sensing coils 182 are fixed to both sides of the bed 31. With the structure shown in Fig. 60C, the drive coil 181 is arranged to both sides of the bed 31. In this case, the same advantages in the case with reference to Figs. 60A and 60B are obtained.

When the drive coil 181 is arranged onto the bed 31 in the vertical direction, the change in planar moving mechanism does not move the sensing coils 182. The three-dimensional magnetic field is easily generated and the position/posture is stably detected.

Next, a description is given of the operation of a magnetic guiding method of the magnetic guiding medical system 180 shown in Fig. 55.

Referring to Fig. 61, upon starting to guide the magnetic field, in step S1, performed is processing for determining a relative position between the magnetic field generating unit 2 and the capsule medical apparatus 72B (abbreviated to a capsule in Fig. 61) having the capsule container 81, serving as an insertion unit in the body cavity of the patient 23. Specifically, before guiding the capsule medical apparatus 72B into the patient 23 on the bed 31, the data is calibrated without the capsule medical apparatus 72B having the marker coil 172a near the system.

In the calibration, the position/posture varying unit 74D changes the position of the bed 31 and, simultaneously, the sensing coils 182 detect the output of drive coil 181 at each position (typical position) thereof.

The outputs of the sensing coils 182 are stored in the calibration data storing unit 185 associated with the position of the bed 31.

After the calibration of the position/posture detecting unit 184, referring to Fig. 61, in step S2, the capsule medical apparatus 72B is guided in the body of the patient 23 on the bed 31. In step S3, the position of the bed 31 is moved (e.g., is moved like a lattice), thereby performing the positional detection at the plurality of positions thereof. In step S4, based on the positional detecting result at the plurality of positions on the bed 31, the current position of the capsule medical apparatus 72B is predicted. In step S5, the position/posture control unit 192 moves the bed 31 via the position/posture varying unit 74D so that the capsule medical apparatus 72B matches the central axis of the magnetic field generating unit 2.

As mentioned above, based on the position/posture detected information of the position/posture detecting unit 184, the control unit 191 and the magnetic field control unit 95 control the bed 31 and the generated magnetic field, thereby stably performing the magnetic guiding operation of the capsule medical apparatus 72B. Specifically, based on calibration data near the current position of the bed 31, a calibration value is obtained by approximation and estimation at the current position.

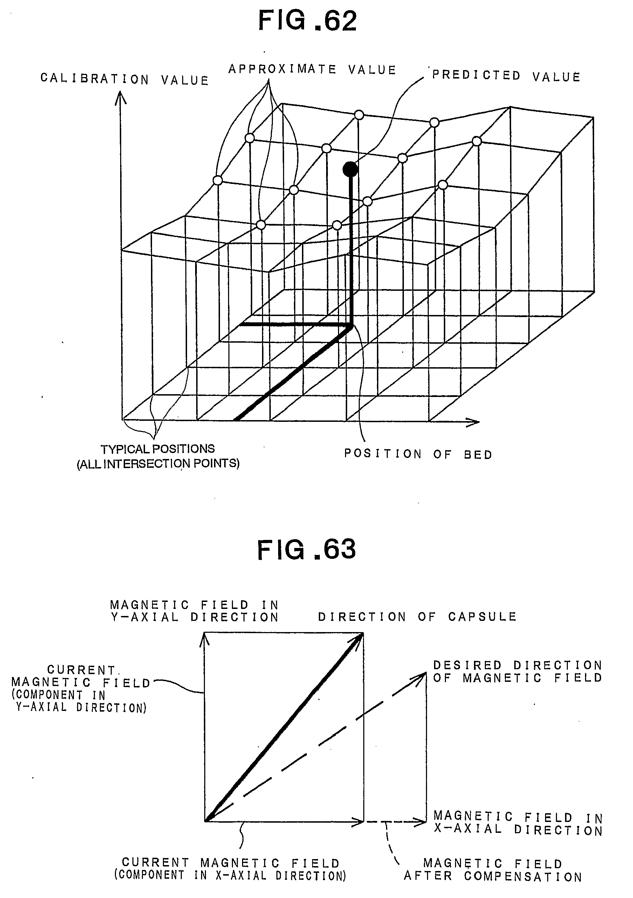

Fig. 62 shows the above-generated calibration values.

Based on the difference between the outputs of the sensing coils 182 and the obtained calibration value, the position/posture detecting unit 184 calculates the position/posture of the capsule medical apparatus 72B.

The bed 31 is moved so that the central axis of the magnetic field generating unit 2 is at the calculated position.

Similarly to the first embodiment, the balance of current flowing to the electromagnets is controlled, based on the information on the obtained position of the capsule medical apparatus 72B and the distance information of the height direction of the magnetic field generating unit 2, so that the magnetic field control unit 95 generates a desired magnetic field at the position of the capsule medical apparatus 72B.

According to the fifth arrangement, with the above-mentioned control operation, the following advantages are obtained.

That is, since the drive coil 181 and the sensing coils 182 are fixed to the magnetic field generating unit 2, the position is not precisely detected without arranging the capsule medical apparatus 72B onto the magnetic field generating unit 2. Then, the bed 31 is moved and the position suitable to the detection is scanned, thereby detecting the position for precise positional detection and setting the magnetic field generating unit 2. From the start timing of the guiding operation, the stable control is possible.

Before generating the magnetic field from the magnetic field generating unit 2, the position/posture of the magnetic field generating unit 2 and the capsule medical apparatus 72B is caused to be within a predetermined range of the relative position/posture, thereby generating the magnetic field suitable to the guiding operation from the start timing of the guiding operation and realizing the stable control operation.

Next, a description is given of a feedback method of the position/posture information from the capsule medical apparatus 72B to the magnetic field control unit 95 according to another modification with reference to Fig. 63. Incidentally, referring to Fig. 63, the capsule medical apparatus is abbreviated to a capsule.

Similarly to the above-mentioned control method, the position of the bed 31 is moved so that the central axis of the magnetic field generating unit 2 matches the capsule medical apparatus 72B. Then, the position/posture is detected again. Here, the obtained posture (direction) of the capsule medical apparatus 72B matches the direction of the magnetic field which is actually generated.

The magnetic field control unit 95 controls and compensates for the current flowing to the electromagnets based on the difference between the direction of the magnetic field to be actually generated and the direction to be originally generated, in order to generate a desired magnetic field.

Further, a description is given of the sequence until starting the guiding operation according to another modification.

Upon guiding the capsule medical apparatus 72B into the patient 23 on the bed 31, the initial position/initial posture of the patient 23 is determined in advance, and the capsule medical apparatus 72B is guided into the patient 23 at the predetermined position/posture. In this case, the bed 31 is marked and the initial position or the initial posture of the patient 23 is determined with the mark. As a mark, a line, serving as the reference may be provided for the bed 31, or laser may be used. Further, the movement of the bed 31 may determine the initial position and the initial posture of the patient 23.

In this case, the bed 31 is moved so that the central axis of the magnetic field generating unit 2 is positioned near the guiding position of the capsule medical apparatus 72B. Further, the position may be detected after moving the magnetic field generating unit 2 and the fine control operation may be performed so that the central axis of the magnetic field generating unit 2 matches the capsule medical apparatus 72B.

Then, the detection of the best position does not need the movement of the bed 31 and, advantageously, the movement to the initial position is possible in a short time. Further, advantageously, the fine control operation sets the position of the bed 31 to the best initial position.

Incidentally, in place of guiding the capsule medical apparatus 72B in the patient 23 on the bed 31, the capsule medical apparatus 72B may be arranged at a predetermined position on the bed 31. In this case, the arrangement position of the capsule medical apparatus 72B may match the central axis of the magnetic field generating unit 2. Further, after the predetermined arrangement position of the capsule medical apparatus 72B matches the central axis of the magnetic field generating unit 2, the positional detection starts and the bed 31 is moved so that the position of the capsule medical apparatus 72B matches the central axis of the magnetic field generating unit 2. In this state, the capsule medical apparatus 72B is guided in the body of the patient 23, thereby starting the guiding operation. Then, the following advantages are obtained.

That is, since the initial position of the capsule medical apparatus 72B is determined with respect to the bed 31, the position of the bed 31 is easily set to the best initial position.

When the drive coil 181 and the sensing coils 182 are fixed to the bed 31 to detect the position, the position is detected when the capsule medical apparatus 72B is guided. The bed may be moved so that central axis of the magnetic field generating unit 2 matches the detected position.

Then, since the position detecting range is wide, the bed 31 is not moved so as to search the position of the capsule medical apparatus 72B. Advantageously, the bed is moved to the initial position in a short time.

Incidentally, according to the fifth arrangement , the magnetic field generating unit 2 is fixed and the bed 31 is moved. The present invention can be applied to the case of moving the magnetic field generating unit 2 according to the first embodiment.

The above-mentioned methods have the similar advantages in the case of another positional detection using electrical waves or the ultrasonic waves.

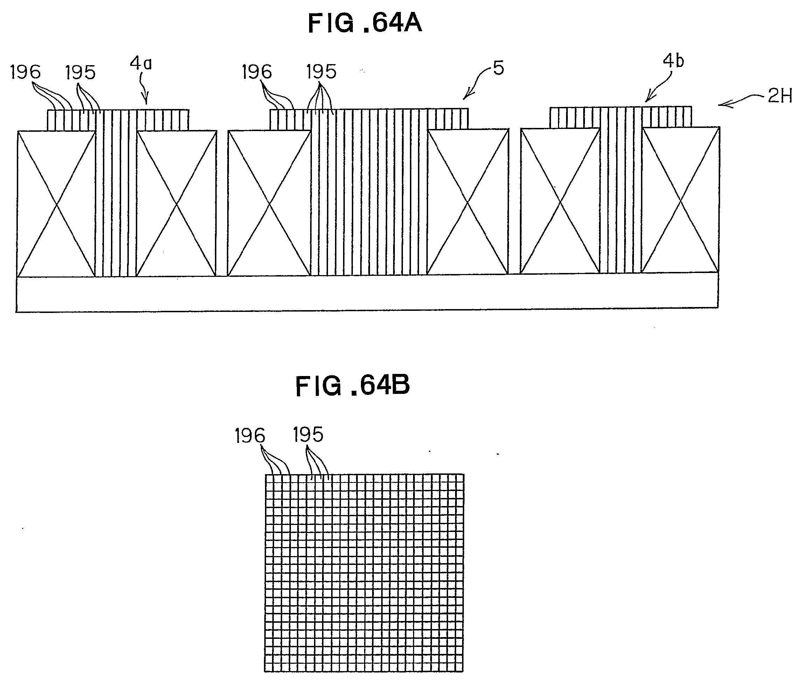

Fig. 64A shows a magnetic field generating unit 2H according to another modification. The magnetic field generating unit 2H has a feature in a ferromagnetic member unit forming the electromagnets.

Referring to Fig. 64B, e.g., a core portion of the electromagnet 5 and a ferromagnetic member (conductor) 195, serving as an auxiliary magnetic-pole portion, are finely partitioned by an insulator 196. The same structure is used for other electromagnets 4a and 4b and the core portion and the ferromagnetic member (conductor) 195, serving as an auxiliary magnetic-pole portion of the electromagnet 3 (not shown in Fig. 64A).

The above-mentioned structure gives the following advantages.

That is, the electromagnet core member and the auxiliary magnetic-pole portion comprise a conductive ferromagnetic member containing iron or Nickel. In this case, the eddy current is generated in alternating magnetic field used by the position detection (position/posture detecting unit 184), thereby influencing on the spatial distribution of alternating magnetic fields. Thus, the positional detecting precision deteriorates. Then, the insulator 196 finely partitions the core portion and the ferromagnetic member 195, serving as an auxiliary magnetic-pole portion, thereby suppressing the eddy current without changing the strength of the generated magnetic field and improving the stability and precision of the position/posture detection. As a consequence, the improvement of the stability and precision of the position/posture for feedback operation raises the precision of the generated magnetic field generated near the insertion unit and enables the stable control operation.

Incidentally, another embodiment structured by partly combining the above-mentioned embodiments belongs to the present invention.

### Industrial Applicability

A living-body inserting medical apparatus, such as a capsule medical apparatus inserted in the body, includes a magnet or the like operated by the magnetic field. The position of the living-body inserting medical apparatus is detected upon magnetically guiding the living-body inserting medical apparatus by a magnetic field generating unit which is arranged to the outside of the body to detect and control the positional movement of the magnetic field generating unit. Thus, even when the living-body inserting medical apparatus is widely moved in the body, the magnetic field for guiding operation is generated using the magnetic field generating unit.

## Claims

1. A magnetic guiding medical system (71) comprising:
a medical apparatus (72) having an insertion unit (81) that is inserted in the body cavity of a living body (23);
a position/posture detecting unit (94) that detects at least one of the position and the posture of the insertion unit (81);
a magnetic field generating unit (2) having at least three electromagnets (3a, 3b, 4a, 4b, 5) that are axial-symmetrically arranged on the substantially same plane and generate magnetic field directions in the orthogonal direction of the plane;
a magnetic field control unit (95) that controls the magnetic field generated by the magnetic field generating unit (2);
a position/posture varying unit (74) that changes a relative position/posture between the magnetic field generating unit (2) and the insertion unit (81) in accordance with information on the position and the posture of the insertion unit (81) obtained by the position/posture detecting unit (94); and
a magnetic field operated unit (88) arranged at the insertion unit (81), wherein
the magnetic field generated by the magnetic field generating unit (2) is applied to the magnetic field operated unit (88), thereby guiding the medical apparatus (72),
**characterized in that**
the insertion unit (81) comprises a capsule container, wherein
the magnetic guiding medical system (71) further comprises:
a receiving antenna unit (73) including a plurality of antennas (73a to 73i) which are arranged two-dimensionally on an upper plane of the magnetic field generating unit (2) and receive wireless signals sent from the medical apparatus (72), wherein a central antenna (73e) is arranged on a central axis (O) of the magnetic field generating unit (72), and
a planar moving mechanism (77) that moves the receiving antenna unit (73) together with the magnetic field generating unit (2) two-dimensionally in parallel with the plane, wherein
the position/posture detecting unit (94) detects the two-dimensional position and posture of the medical apparatus (72) by input of the signals received by the receiving antenna unit (73), and
the magnetic field generating unit (2) and the receiving antenna unit (73) are controlled to be moved two-dimensionally by the planar moving mechanism (77) so that a center of the magnetic field generating unit (2) matches the detected two-dimensional position of the medical apparatus (72).

2. The magnetic guiding medical system according to Claim 1, wherein the magnetic field generating unit (2) comprises:
a first electromagnet unit comprising two electromagnets (3a, 3b); and
a second electromagnet unit comprising one electromagnet (5),
the two electromagnets (3a, 3b) forming the first electromagnet unit are arranged on the plane at a predetermined interval to have the magnetizing directions opposite to each other, and
the one electromagnet (5) forming the second electromagnet unit is arranged in the substantially center of the two electromagnets (3a, 3b) forming the first electromagnet unit.

3. The magnetic guiding medical system according to Claim 2, wherein the magnetic field generating unit (2) further comprises:
a third electromagnet unit comprising two electromagnets (4a, 4b), and
the two electromagnets (4a, 4b) forming the third electromagnet unit are arranged on the plane at a predetermined interval to have the magnetizing directions opposite to each other, and
the third electromagnet unit and the first electromagnet unit are substantially orthogonal to each other on the plane, and
a central portion of the third electromagnet unit is arranged to substantially match the second electromagnet unit.

4. The magnetic guiding medical system according to Claim 2, wherein the magnetic field control unit (95) comprises a relative position control unit that changes the relative position of the second electromagnet unit and other electromagnet unit in the direction vertical to the plane based on the position information of the insertion unit (81) obtained by the position/posture detecting unit (94).

5. The magnetic guiding medical system according to Claim 2, wherein the magnetic field control unit (95) comprises a rotating mechanism (146) that rotates the magnetic field generating unit (2) on the plane.

6. The magnetic guiding medical system according to Claim 1, wherein the magnetic field generating unit (2) comprises:
a first electromagnet unit comprising two electromagnets (3a, 3b); and
a third electromagnet unit comprising two electromagnets (4a, 4b), the two electromagnets (3a, 3b) forming the first electromagnet unit are arranged on the plane at a predetermined interval to have magnetizing directions opposite to each other,
the two electromagnets (4a, 4b) forming the third electromagnet unit are arranged on the plane at a predetermined interval to have the magnetizing directions opposite to each other, and
the first electromagnet unit and the third electromagnet unit are substantially orthogonal to each other on the plane, and central portions thereof are arranged to match each other.

7. The magnetic guiding medical system according to Claim 1, wherein the magnetic field generating unit (2) further comprises at least one electromagnet facing at least one of the electromagnets so as to sandwich the insertion unit (81).

8. The magnetic guiding medical system according to Claim 1, wherein the position/posture varying unit (74) comprises a planar moving mechanism that relatively planar-moves the magnetic field generating unit (2) and the living body (23) on the plane.

9. The magnetic guiding medical system according to Claim 8, wherein the position/posture varying unit (74) comprises:
an in-planar-position control unit (78) that controls the planar moving mechanism based on the positional information of the inserting unit (81) obtained by the position/posture detecting unit (94) so that the insertion unit (81) exists on the symmetrical axis of the magnetic field generating unit (2).

10. The magnetic guiding medical system according to Claim 1, wherein the position/posture varying unit (74) comprises a vertical moving mechanism (142) that relatively moves the magnetic field generating unit (2) and the living body (23) in the vertical direction with respect to the plane.

11. The magnetic guiding medical system according to Claim 10, wherein the position/posture varying unit (74) comprises a vertical-position control unit that controls the vertical moving mechanism (142) based on the positional information of the insertion unit (81) which is obtained by the position/posture detecting unit (94) so as to set, to be constant, the distance between the magnetic field generating unit (2) and the insertion unit (81) in the vertical direction with respect to the plane.

12. The magnetic guiding medical system according to Claim 1, wherein the magnetic field control unit (95) comprises an electromagnet current control unit (96) that controls current flowing to the plurality of electromagnets, and the electromagnet current control unit (96) controls the current flowing to the electromagnet so as to generate the magnetic field in an arbitrary direction at the position of the insertion unit (81) detected by the position/posture detecting unit (94).

13. The magnetic guiding medical system according to Claim 1, wherein the magnetic field control unit (95) comprises a generated magnetic field storing unit (97) that stores information of the current flowing to each of the plurality of electromagnets and information of the generated magnetic field at positions on the symmetrical axis in association with each other, and
the magnetic field control unit (95) controls the magnetic field generating unit (2) in accordance with the positional information of the insertion unit (81) obtained by the position/posture detecting unit (94), the current information of the electromagnets stored by the generated magnetic field storing unit (97), and information of the generated magnetic field on the symmetrical axis.

14. The magnetic guiding medical system according to Claim 1, wherein the insertion unit comprises a magnetic field measurement unit, and the magnetic field control unit controls the magnetic field generated by the magnetic field generating unit in accordance with the position and the posture of the insertion unit which is obtained by the position/posture detecting unit and the magnetic field obtained by the magnetic field measurement unit.

15. The magnetic guiding medical system according to Claim 1, wherein the magnetic field control unit (95) controls the magnetic field generating unit (2) based on the difference between the direction of the magnetic field to be generated and the posture of the insertion unit obtained by the position/posture detecting unit (94).

16. The magnetic guiding medical system according to Claim 1, wherein the position/posture detecting unit (94) comprises an electromagnetic-wave generating unit arranged to the insertion unit (81) that generates electromagnetic waves and at least one receiving unit that receives the electromagnetic waves outside the living body (23), and at least one of the position and the posture is detected based on the strength of the electromagnetic waves received by the receiving unit.

17. The magnetic guiding medical system according to Claim 16 wherein a frame that changes the position thereof relative to the magnetic field generating unit (2) by the operation of the position/posture varying unit (74) is provided, and the receiving unit is fixed to the frame.

18. The magnetic guiding medical system according to Claim 16, wherein the receiving unit is fixed relatively to the magnetic field generating unit (2).

19. The magnetic guiding medical system according to Claim 1, wherein the position/posture detecting unit (94) comprises:
a marker coil (172a) arranged to the insertion unit (81);
a plurality of magnetic sensors (126) that are arranged outside the living body (23) and detect the strength of the magnetic field generated by the marker coil (172a); and
a position/posture calculating unit for calculating at least one of the position and the posture of the insertion unit (81) based on the magnetic field detected by the plurality of magnetic sensors (126).

20. The magnetic guiding medical system according to Claim 19, wherein the magnetic sensor (126) is fixed relatively to the magnetic field generating unit (2).

21. The magnetic guiding medical system according to Claim 19, wherein a frame that changes a relative position thereof to the magnetic field generating unit (2) by the operation of the position/posture varying unit (74) is provided, and the magnetic sensor (126) is fixed to the frame.

22. The magnetic guiding medical system according to Claim 19, wherein the position/posture detecting unit (94) further comprises a drive coil (181) that is arranged outside the living body (23) and generates a variable magnetic field so that the marker coil (172a) generates an induction magnetic field.

23. The magnetic guiding medical system according to Claim 22, wherein the position/posture detecting unit (94) further comprises:
a calibration data storing unit (185) that stores calibration data, serving as outputs of a plurality of magnetic sensors (126) when only the variable magnetic field generated by the drive coil (181) is applied to the plurality of magnetic sensors (126), and the position/posture of the insertion unit (81) is calculated based on the outputs of the plurality of magnetic sensors (126) and the calibration data stored in the calibration data storing unit (185), upon applied, to the plurality of magnetic sensors (126), the variable magnetic field generated by the drive coil (181) and the induction magnetic field generated by the marker coil (172a) induced by the variable magnetic field.

24. The magnetic guiding medical system according to Claim 22, wherein the calibration data storing unit (185) stores a plurality of positions and postures of the position/posture varying unit (74) and the calibration data in accordance with the position and posture in association with each other therebetween, the position/posture calculating unit (185) obtains the calibration data in accordance with the positions and postures of the position/posture varying unit (74) based on the calibration data stored in the calibration data storing unit (185), and at least one of the position and the posture of the insertion unit (81) is calculated based on the output of the magnetic sensor (126) and the calibration data obtained by the position/posture calculating unit.

25. The magnetic guiding medical system according to Claim 22, wherein the drive coil (181) is fixed relatively to the magnetic field generating unit (2).

26. The magnetic guiding medical system according to Claim 22, wherein a frame that changes the position thereof relative to the magnetic field generating unit (2) by the operation of the position/posture varying unit (74) is provided, and the drive coil (181) is fixed to the frame.

27. The magnetic guiding medical system according to Claim 1, wherein a core of at least any one of the three electromagnets (3a, 3b, 4a, 4b, 5) comprises a ferromagnetic member, and the cross-sectional area on the plane vertical to the magnetizing direction of the core is maximum on the end surface near the living body (23).

## Patentansprüche

1. Medizinisches magnetisches Führungssystem (71), das umfasst:
ein medizinisches Gerät (72) mit einer Einführeinheit (81), die in den Körperhohlraum eines lebenden Körpers (23) eingeführt ist;
eine Positions/Lage-Erfassungseinheit (94), die die Position und/oder die Lage der Einführeinheit (81) erfasst;
eine Magnetfelderzeugungseinheit (2) mit mindestens drei Elektromagneten (3a, 3b, 4a, 4b, 5), die achsensymmetrisch auf der im Wesentlichen gleichen Ebene angeordnet sind und Magnetfeldrichtungen in der orthogonal Richtung der Ebene erzeugen;
eine Magnetfeldsteuereinheit (95), die das von der Magnetfelderzeugungseinheit (2) erzeugte Magnetfeld steuert;
eine Positions/Lage-Variationseinheit (74), die eine relative Position/Lage zwischen der Magnetfelderzeugungseinheit (2) und der Einführeinheit (81) gemäß einer von der Positions/Lage-Erfassungseinheit (94) erhaltenen Information über die Position und die Lage der Einführeinheit (81) verändert; und
eine magnetfeldbetätigte Einheit (88), die in der Einführeinheit (81) angeordnet ist, wobei
das von der Magnetfelderzeugungseinheit (2) erzeugte Magnetfeld an die magnetfeldbetätigte Einheit (88) angelegt wird, wodurch das medizinische Gerät (72) geführt wird,
**dadurch gekennzeichnet, dass**
die Einführeinheit (81) ein Kapselgehäuse umfasst, wobei
das medizinische magnetische Führungssystem (71) ferner umfasst:
eine Empfangsantenneneinheit (73) mit einer Mehrzahl von Antennen (73a bis 73i), die zweidimensional auf einer oberen Ebene der Magnetfelderzeugungseinheit (2) angeordnet sind und drahtlos von dem medizinischen Gerät (72) gesendete Signale empfangen, wobei eine zentrale Antenne (73e) auf einer zentralen Achse (O) der Magnetfelderzeugungseinheit (72) angeordnet ist, und
einen ebenen Bewegungsmechanismus (77), der die Empfangsantenneneinheit (73) gemeinsam mit der Magnetfelderzeugungseinheit (2) zweidimensional parallel mit der Ebene bewegt, wobei
die Positions/Lage-Erfassungseinheit (94) durch eine Eingabe der von der Empfangsantenneneinheit (73) empfangenen Signale die zweidimensionale Position und Lage des medizinischen Geräts (72) erfasst, und
die Magnetfelderzeugungseinheit (2) und die Empfangsantenneneinheit (73) gesteuert werden, um durch den ebenen Bewegungsmechanismus (77) zweidimensional so bewegt zu werden, dass ein Zentrum der Magnetfelderzeugungseinheit (2) mit der erfassten zweidimensionalen Position des medizinischen Geräts (72) zusammenpasst.

2. Medizinisches magnetisches Führungssystem nach Anspruch 1, bei dem die Magnetfelderzeugungseinheit (2) umfasst:
eine erste Elektromagneteinheit, die zwei Elektromagnete (3a, 3b) umfasst; und
eine zweite Elektromagneteinheit, die einen Elektromagneten (5) umfasst, wobei die die erste Elektromagneteinheit bildenden zwei Elektromagnete (3a, 3b) auf der Ebene in einem vorbestimmten Intervall angeordnet sind, um entgegengesetzte Magnetisierungsrichtungen zu haben, und
der die zweite Elektromagneteinheit bildende eine Elektromagnet (5) im Wesentlichen im Zentrum der die erste Elektromagneteinheit bildenden zwei Elektromagnete (3a, 3b) angeordnet ist.

3. Medizinisches magnetisches Führungssystem nach Anspruch 2, bei dem die Magnetfelderzeugungseinheit (2) ferner umfasst:
eine dritte Elektromagneteinheit, die zwei Elektromagnete (4a, 4b) umfasst; und
wobei die die dritte Elektromagneteinheit bildenden zwei Elektromagnete (4a, 4b) auf der Ebene in einem vorbestimmten Intervall angeordnet sind, um entgegengesetzte Magnetisierungsrichtungen zu haben, und
die dritte Elektromagneteinheit und die erste Elektromagneteinheit im Wesentlichen orthogonal zueinander auf der Ebene liegen, und
ein zentraler Abschnitt der dritten Elektromagneteinheit so angeordnet ist, dass er im Wesentlichen mit der zweiten Elektromagneteinheit zusammenpasst.

4. Medizinisches magnetisches Führungssystem nach Anspruch 2, bei dem die Magnetfeldsteuereinheit (95) eine Relativpositionssteuereinheit umfasst, die auf der Basis der von der Positions/Lage-Erfassungseinheit (94) erhaltenen Positionsinformation der Einführeinheit (81) die relative Position der zweiten Elektromagneteinheit und eine anderen Elektromagneteinheit in der Richtung vertikal zu der Ebene verändert.

5. Medizinisches magnetisches Führungssystem nach Anspruch 2, bei dem die Magnetfeldsteuereinheit (95) einen Drehmechanismus (146) umfasst, der die Magnetfelderzeugungseinheit (2) auf der Ebene dreht.

6. Medizinisches magnetisches Führungssystem nach Anspruch 1, bei dem die Magnetfelderzeugungseinheit (2) umfasst:
eine erste Elektromagneteinheit, die zwei Elektromagnete (3a, 3b) umfasst; und
eine dritte Elektromagneteinheit, die zwei Elektromagnete (4a, 4b) umfasst, wobei die die erste Elektromagneteinheit bildenden zwei Elektromagnete (3a, 3b) auf der Ebene in einem vorbestimmten Intervall angeordnet sind, um entgegengesetzte Magnetisierungsrichtungen zu haben,
die die dritte Elektromagneteinheit bildenden zwei Elektromagnete (4a, 4b) auf der Ebene in einem vorbestimmten Intervall angeordnet sind, um entgegengesetzte Magnetisierungsrichtungen zu haben, und
die erste Elektromagneteinheit und die dritte Elektromagneteinheit im Wesentlichen orthogonal zueinander auf der Ebene liegen, und zentrale Abschnitte davon so angeordnet sind, dass sie zusammenpassen.

7. Medizinisches magnetisches Führungssystem nach Anspruch 1, bei dem die Magnetfelderzeugungseinheit (2) ferner mindestens einen Elektromagneten umfasst, der mindestens einem der Elektromagneten gegenüber liegt, um die Einführeinheit (81) dazwischen zu positionieren.

8. Medizinisches magnetisches Führungssystem nach Anspruch 1, bei dem die Positions/Lage-Variationseinheit (74) einen ebenen Bewegungsmechanismus umfasst, der die Magnetfelderzeugungseinheit (2) und den lebenden Körper (23) relativ zueinander eben auf der Ebene bewegt.

9. Medizinisches magnetisches Führungssystem nach Anspruch 8, bei dem die Positions/Lage-Variationseinheit (74) umfasst:
eine Einheit (78) zur Steuerung einer Position in der Ebene, die den ebenen Bewegungsmechanismus auf der Basis der von der Positions/Lage-Erfassungseinheit (94) erhaltenen Positionsinformation der Einführeinheit (81) so steuert, dass die Einführeinheit (81) auf der Symmetrieachse der Magnetfelderzeugungseinheit (2) existiert.

10. Medizinisches magnetisches Führungssystem nach Anspruch 1, bei dem die Positions/Lage-Variationseinheit (74) einen vertikalen Bewegungsmechanismus (142) umfasst, der die Magnetfelderzeugungseinheit (2) und den lebenden Körper (23) relativ zueinander in der vertikalen Richtung bezüglich der Ebene bewegt.

11. Medizinisches magnetisches Führungssystem nach Anspruch 10, bei dem die Positions/Lage-Variationseinheit (74) eine Einheit zur Steuerung einer vertikalen Position umfasst, die den vertikalen Bewegungsmechanismus (142) auf der Basis der von der Positions/Lage-Erfassungseinheit (94) erhaltenen Positionsinformation der Einführeinheit (81) so steuert, dass der Abstand zwischen der Magnetfelderzeugungseinheit (2) und der Einführeinheit (1) in der vertikalen Richtung bezüglich der Ebene als konstant eingestellt wird.

12. Medizinisches magnetisches Führungssystem nach Anspruch 1, bei dem die Magnetfeldsteuereinheit (95) eine Elektromagnetstromsteuereinheit (96) umfasst, die einen zu der Mehrzahl von Elektromagneten fließenden Strom steuert und die Elektromagnetstromsteuereinheit (96) den zu den Elektromagneten fließenden Strom steuert, um an der von der Positions/Lage-Erfassungseinheit (94) erfassten Position der Einführeinheit (81) das Magnetfeld in einer beliebigen Richtung zu erzeugen.

13. Medizinisches magnetisches Führungssystem nach Anspruch 1, bei dem die Magnetfeldsteuereinheit (95) eine Einheit (97) zum Speichern eines erzeugten Magnetfelds umfasst, die eine Information des zu jedem der Mehrzahl von Elektromagneten fließenden Stroms und eine Information des an Positionen auf der Symmetrieachse erzeugten Magnetfelds in Bezug zueinander speichert, und
die Magnetfeldsteuereinheit (95) die Magnetfelderzeugungseinheit (2) gemäß der von der Positions/Lage-Erfassungseinheit (94) erhaltenen Positionsinformation der Einführeinheit (81), der von der Einheit (97) zum Speichern eines erzeugten Magnetfelds gespeicherten Strominformation der Elektromagneten und der Information des auf der Symmetrieachse erzeugten Magnetfelds steuert.

14. Medizinisches magnetisches Führungssystem nach Anspruch 1, bei dem die Einführeinheit eine Magnetfeldmesseinheit umfasst und die Magnetfeldsteuereinheit das von der Magnetfelderzeugungseinheit erzeugte Magnetfeld gemäß der von der Positions/Lage-Erfassungseinheit erhaltenen Position und Lage der Einführeinheit und dem von der Magnetfeldmesseinheit erhaltenen Magnetfeld steuert.

15. Medizinisches magnetisches Führungssystem nach Anspruch 1, bei dem die Magnetfeldsteuereinheit (95) die Magnetfelderzeugungseinheit (2) auf der Basis der Differenz zwischen der Richtung des zu erzeugenden Magnetfelds und der von der Positions/Lage-Erfassungseinheit (94) erhaltenen Lage der Einführeinheit steuert.

16. Medizinisches magnetisches Führungssystem nach Anspruch 1, bei dem die Positions/Lage-Erfassungseinheit (94) eine zu der Einführeinheit (81) angeordnete Einheit zur Erzeugung einer elektromagnetischen Welle, die elektromagnetische Wellen erzeugt, und mindestens eine Empfangseinheit, die die elektromagnetischen Wellen außerhalb des lebenden Körpers (23) empfängt, umfasst, und die Position und/oder die Lage auf der Basis der Stärke der von der Empfangseinheit empfangenen elektromagnetischen Wellen erfasst wird.

17. Medizinisches magnetisches Führungssystem nach Anspruch 16, bei dem ein Rahmen, der durch den Betrieb der Positions/Lage-Variationseinheit (74) seine Position relativ zu der Magnetfelderzeugungseinheit (2) verändert, vorgesehen ist und die Empfangseinheit an dem Rahmen befestigt ist.

18. Medizinisches magnetisches Führungssystem nach Anspruch 16, bei dem die Empfangseinheit relativ zu der Magnetfelderzeugungseinheit (2) befestigt ist.

19. Medizinisches magnetisches Führungssystem nach Anspruch 16, bei dem die Positions/Lage-Erfassungseinheit (94) umfasst:
eine zu der Einführeinheit (81) angeordnete Markierungsspule (172a);
eine Mehrzahl von magnetischen Sensoren (126), die außerhalb des lebenden Körpers (23) angeordnet sind und die Stärke des von der Markierungsspule (172a) erzeugten Magnetfelds erfassen; und
eine Positions/Lage-Berechnungseinheit zum Berechnen der Position und/oder der Lage der Einführeinheit (81) auf der Basis des von der Mehrzahl von magnetischen Sensoren (126) erfassten Magnetfelds.

20. Medizinisches magnetisches Führungssystem nach Anspruch 19, bei dem der magnetische Sensor (126) relativ zu der Magnetfelderzeugungseinheit (2) befestigt ist.

21. Medizinisches magnetisches Führungssystem nach Anspruch 19, bei dem ein Rahmen, der durch den Betrieb der Positions/Lage-Variationseinheit (74) seine Position relativ zu der Magnetfelderzeugungseinheit (2) verändert, vorgesehen ist und der Magnetfeldsensor (126) an dem Rahmen befestigt ist.

22. Medizinisches magnetisches Führungssystem nach Anspruch 16, bei dem die Positions/Lage-Erfassungseinheit (94) ferner eine Antriebsspule (181) umfasst, die außerhalb des lebenden Körpers (23) angeordnet ist und einen magnetisches Wechselfeld erzeugt, so dass die Markierungsspule (172a) ein Induktionsmagnetfeld erzeugt.

23. Medizinisches magnetisches Führungssystem nach Anspruch 22, bei dem die Positions/Lage-Erfassungseinheit (94) ferner umfasst:
eine Kalibrierungsdatenspeichereinheit (185), die Kalibrierungsdaten speichert, die als Ausgaben einer Mehrzahl von magnetischen Sensoren (120) dienen, wenn nur das von der Antriebsspule (181) erzeugte magnetische Wechselfeld an die Mehrzahl von magnetischen Sensoren (126) angelegt ist, und die Position/Lage der Einführeinheit (81) auf der Basis der Ausgaben der Mehrzahl von magnetischen Sensoren (126) und der in der Kalibrierungsdatenspeichereinheit (185) gespeicherten Daten berechnet wird/werden, wenn an die Mehrzahl von magnetischen Sensoren (126) das von der Antriebsspule (181) erzeugte magnetische Wechselfeld und das von dem magnetischen Wechselfeld induzierte, von der Markierungsspule (172a) erzeugte Induktionsmagnetfeld angelegt wird.

24. Medizinisches magnetisches Führungssystem nach Anspruch 22, bei dem die Kalibrierungsdatenspeichereinheit (185) eine Mehrzahl von Positionen und Lagen der Positions/Lage-Variationseinheit (74) und die Kalibrierungsdaten gemäß der Position und Lage im Zusammenhang miteinander speichert, die Positions/Lage-Berechnungseinheit (185) die Kalibrierungsdaten gemäß der Positionen und Lagen der Positions/Lage-Variationseinheit (74) auf der Basis der in der Kalibrierungsdatenspeichereinheit (185) gespeicherten Kalibrierungsdaten erhält und die Position und/oder die Lage der Einführeinheit (81) auf der Basis der Ausgabe des magnetischen Sensors (126) und der von der Positions/Lage-Berechnungseinheit erhaltenen Kalibrierungsdaten berechnet wird.

25. Medizinisches magnetisches Führungssystem nach Anspruch 22, bei dem die Antriebsspule (181) relativ zu der Magnetfelderzeugungseinheit (2) befestigt ist.

26. Medizinisches magnetisches Führungssystem nach Anspruch 22, bei dem ein Rahmen, der durch den Betrieb der Positions/Lage-Variationseinheit (74) seine Position relativ zu der Magnetfelderzeugungseinheit (2) verändert, vorgesehen ist und die Antriebsspule (181) an dem Rahmen befestigt ist.

27. Medizinisches magnetisches Führungssystem nach Anspruch 1, bei dem ein Kern mindestens eines beliebigen der drei Elektromagneten (3a, 3b, 4a, 4b, 5) ein ferromagnetisches Element umfasst und die Querschnittsfläche auf der Ebene vertikal zur Magnetisierungsrichtung des Kerns an der Stirnfläche in der Nähe des lebenden Körpers (23) maximal ist.

## Revendications

1. Système (71) médical de guidage magnétique comprenant :
un appareil médical (72) ayant une unité (81) d'insertion qui est insérée dans la cavité corporelle d'un corps vivant (23) ;
une unité (94) de détection de position/posture qui détecte au moins une da la position et de la posture de l'unité (81) d'insertion ;
une unité (2) de génération de champ magnétique ayant au moins trois électroaimants (3a, 3b, 4a, 4b, 5) qui sont agencés axialement symétriquement sur sensiblement le même plan et génèrent des sens de champ magnétique dans le sens orthogonal du plan ;
une unité (95) de commande de champ magnétique qui commande le champ magnétique généré par l'unité (2) de génération de champ magnétique ;
une unité (74) de variation de position/posture qui change une position/posture relatives entre l'unité (2) de génération de champ magnétique et l'unité (81) d'insertion en fonction d'une information sur la position et la posture de l'unité (81) d'insertion obtenue par l'unité (94) de détection de position/posture ; et
une unité (88) opérée par champ magnétique agencée au niveau de l'unité (81) d'insertion, dans lequel
le champ magnétique généré par l'unité (2) de génération de champ magnétique est appliqué à l'unité (88) opérée par champ magnétique, guidant ainsi l'appareil médical (72),
**caractérisé en ce que**
l'unité (81) d'insertion comprend un contenant de type capsule, dans lequel le système (71) médical de guidage magnétique comprend en outre :
une unité (73) d'antennes de réception incluant une pluralité d'antennes (73a à 73i) qui sont agencées en deux dimensions sur un plan supérieur de l'unité (2) de génération de champ magnétique et reçoivent des signaux sans fil envoyés depuis l'appareil médical (72), dans lequel une antenne centrale (73e) est agencée sur un axe central (O) de l'unité (72) de génération de champ magnétique, et
un mécanisme (77) de déplacement plan qui déplace l'unité (73) d'antennes de réception en même temps que l'unité (2) de génération de champ magnétique en deux dimensions en parallèle avec le plan, dans lequel
l'unité (94) de détection de position/posture détecte la position et la posture en deux dimensions de l'appareil médical (72) par entrée des signaux reçus par l'unité (73) d'antennes de réception, et
l'unité (2) de génération de champ magnétique et l'unité (73) d'antennes de réception sont commandées pour être déplacées en deux dimensions par le mécanisme (77) de déplacement plan de telle manière qu'un centre de l'unité (2) de génération de champ magnétique correspond à la position en deux dimensions détectée de l'appareil médical (72).

2. Système médical de guidage magnétique selon la revendication 1, dans lequel l'unité (2) de génération de champ magnétique comprend :
une première unité d'électroaimants comprenant deux électroaimants (3a, 3b) ; et
une deuxième unité d'électroaimant comprenant un électroaimant (5),
les deux électroaimants (3a, 3b) formant la première unité d'électroaimants sont agencés sur le plan à un intervalle prédéterminé pour avoir les sens de magnétisation opposés l'un à l'autre, et
l'électroaimant (5) formant la deuxième unité d'électroaimant est agencé sensiblement dans le centre des deux électroaimants (3a, 3b) formant la première unité d'électroaimants.

3. Système médical de guidage magnétique selon la revendication 2, dans lequel l'unité (2) de génération de champ magnétique comprend en outre :
une troisième unité d'électroaimants comprenant deux électroaimants (4a, 4b), et
les deux électroaimants (4a, 4b) formant la troisième unité d'électroaimants sont agencés sur le plan à un intervalle prédéterminé pour avoir les sens de magnétisation opposés l'un à l'autre, et
la troisième unité d'électroaimants et la première unité d'électroaimants sont sensiblement orthogonales l'une à l'autre sur le plan, et
une partie centrale de la troisième unité d'électroaimants est agencée pour sensiblement correspondre à la deuxième unité d'électroaimants.

4. Système médical de guidage magnétique selon la revendication 2, dans lequel l'unité (95) de commande de champ magnétique comprend une unité de commande de position relative qui change la position relative de la deuxième unité d'électroaimant et d'une autre unité d'électroaimants dans le sens vertical par rapport au plan sur la base de l'information de position de l'unité (81) d'insertion obtenue par l'unité (94) de détection de position/posture.

5. Système médical de guidage magnétique selon la revendication 2, dans lequel l'unité (95) de commande de champ magnétique comprend un mécanisme (146) de rotation qui fait tourner l'unité (2) de génération de champ magnétique sur le plan.

6. Système médical de guidage magnétique selon la revendication 1, dans lequel l'unité (2) de génération de champ magnétique comprend :
une première unité d'électroaimants comprenant deux électroaimants (3a, 3b) ; et
une troisième unité d'électroaimants comprenant deux électroaimants (4a, 4b), les deux électroaimants (3a, 3b) formant la première unité d'électroaimants sont agencés sur le plan à un intervalle prédéterminé pour avoir les sens de magnétisation opposés l'un à l'autre,
les deux électroaimants (4a, 4b) formant la troisième unité d'électroaimants sont agencés sur le plan à un intervalle prédéterminé pour avoir les sens de magnétisation opposés l'un à l'autre, et
la première unité d'électroaimants et la troisième unité d'électroaimants sont sensiblement orthogonales l'une à l'autre sur le plan, et des parties centrales de celles-ci sont agencées de façon à correspondre l'une à l'autre.

7. Système médical de guidage magnétique selon la revendication 1, dans lequel l'unité (2) de génération de champ magnétique comprend en outre au moins un électroaimant faisant face à un des électroaimants de façon à prendre en sandwich l'unité (81) d'insertion.

8. Système médical de guidage magnétique selon la revendication 1, dans lequel l'unité (74) de variation de position/posture comprend un mécanisme de déplacement plan qui déplace de façon relativement plane l'unité (2) de génération de champ magnétique et le corps vivant (23) sur le plan.

9. Système médical de guidage magnétique selon la revendication 8, dans lequel l'unité (74) de variation de position/posture comprend :
une unité (78) de commande de position dans le plan qui commande le mécanisme de déplacement plan sur la base de l'information positionnelle de l'unité (81) d'insertion obtenue par l'unité (94) de détection de position/posture de telle manière que l'unité (81) d'insertion existe sur l'axe symétrique de l'unité (2) de génération de champ magnétique.

10. Système médical de guidage magnétique selon la revendication 1, dans lequel l'unité (74) de variation de position/posture comprend un mécanisme (142) de déplacement vertical qui déplace relativement l'unité (2) de génération de champ magnétique et le corps vivant (23) dans le sens vertical par rapport au plan.

11. Système médical de guidage magnétique selon la revendication 10, dans lequel l'unité (74) de variation de position/posture comprend une unité de commande de position verticale qui commande le mécanisme (142) de déplacement vertical sur la base de l'information positionnelle de l'unité (81) d'insertion qui est obtenue par l'unité (94) de détection de position/posture de façon à fixer, pour qu'elle soit constante, la distance entre l'unité (2) de génération de champ magnétique et l'unité (81) d'insertion dans le sens vertical par rapport au plan.

12. Système médical de guidage magnétique selon la revendication 1, dans lequel l'unité (95) de commande de champ magnétique comprend une unité (96) de commande de courant d'électroaimants qui commande un courant allant jusqu'à la pluralité d'électroaimants, et l'unité (96) de commande de courant d'électroaimants commande le courant jusqu'à l'électroaimant de façon à générer le champ magnétique dans un sens arbitraire à la position de l'unité (81) d'insertion détectée par l'unité (94) de détection de position/posture.

13. Système médical de guidage magnétique selon la revendication 1, dans lequel l'unité (95) de commande de champ magnétique comprend une unité (97) de stockage de champ magnétique généré qui stocke des informations du courant allant jusqu'à chacun de la pluralité d'électroaimants et des informations du champ magnétique généré à des positions sur l'axe symétrique en association les unes avec les autres, et
l'unité (95) de commande de champ magnétique commande l'unité (2) de génération de champ magnétique en fonction de l'information positionnelle de l'unité (81) d'insertion obtenue par l'unité (94) de détection de position/posture, des informations de courant des électroaimants stockées par l'unité (97) de stockage de champ magnétique généré, et des informations du champ magnétique généré sur l'axe symétrique.

14. Système médical de guidage magnétique selon la revendication 1, dans lequel l'unité d'insertion comprend une unité de mesure de champ magnétique, et l'unité de commande de champ magnétique commande la champ magnétique généré par l'unité de génération de champ magnétique en fonction de la position et de la posture de l'unité d'insertion qui sont obtenues par l'unité de détection de position/posture et du champ magnétique obtenu par l'unité de mesure de champ magnétique.

15. Système médical de guidage magnétique selon la revendication 1, dans lequel l'unité (95) de commande de champ magnétique commande l'unité (2) de génération de champ magnétique sur la base de la différence entre le sens du champ magnétique devant être généré et la posture de l'unité d'insertion obtenue par l'unité (94) de détection de position/posture.

16. Système médical de guidage magnétique selon la revendication 1, dans lequel l'unité (94) de détection de position/posture comprend une unité de génération d'ondes électromagnétiques agencée sur l'unité (81) d'insertion qui génère des ondes électromagnétiques et au moins une unité de réception qui reçoit les ondes électromagnétiques à l'extérieur du corps vivant (23), et au moins une de la position et de la posture est détectée sur la base de la force des ondes électromagnétiques reçues par l'unité de réception.

17. Système médical de guidage magnétique selon la revendication 16, dans lequel un cadre qui change la position de celui-ci relativement à l'unité (2) de génération de champ magnétique par l'opération de l'unité (74) de variation de position/posture est prévu, et l'unité de réception est fixée au cadre.

18. Système médical de guidage magnétique selon la revendication 16, dans lequel l'unité de réception est fixe relativement à l'unité (2) de génération de champ magnétique.

19. Système médical de guidage magnétique selon la revendication 1, dans lequel l'unité (94) de détection de position/posture comprend :
une bobine de marquage (172a) agencée sur l'unité (81) d'insertion ;
une pluralité de capteurs magnétiques (126) qui sont agencés à l'extérieur du corps vivant (23) et détectent la force du champ magnétique généré par la bobine de marquage (172a) ; et
une unité de calcul de position/posture pour calculer au moins une de la position et de la posture de l'unité (81) d'insertion sur la base du champ magnétique détecté par la pluralité de capteurs magnétiques (126).

20. Système médical de guidage magnétique selon la revendication 19, dans lequel le capteur magnétique (126) est fixe par rapport à l'unité (2) de génération de champ magnétique.

21. Système médical de guidage magnétique selon la revendication 19, dans lequel un cadre qui change une position relative de celui-ci par rapport à l'unité (2) de génération de champ magnétique par l'opération de l'unité (74) de variation de position/posture est prévu, et le capteur magnétique (126) est fixé au cadre.

22. Système médical de guidage magnétique selon la revendication 19, dans lequel l'unité (94) de détection de position/posture comprend en outre une bobine d'excitation (181) qui est agencée à l'extérieur du corps vivant (23) et génère un champ magnétique variable de telle sorte que la bobine de marquage (172a) génère un champ magnétique d'induction.

23. Système médical de guidage magnétique selon la revendication 22, dans lequel l'unité (94) de détection de position/posture comprend en outre :
une unité (185) de stockage de données d'étalonnage qui stocke des données d'étalonnage, servant de sorties d'une pluralité de capteurs magnétiques (126) lorsque seul le champ magnétique variable généré par la bobine d'excitation (181) est appliqué à la pluralité de capteurs magnétiques (126), et la position/posture de l'unité (81) d'insertion est calculée sur la base des sorties de la pluralité de capteurs magnétiques (126) et des données d'étalonnage stockées dans l'unité (185) de stockage de données d'étalonnage, lorsqu'elles sont appliquées à la pluralité de capteurs magnétiques (126), du champ magnétique variable généré par la bobine d'excitation (181) et du champ magnétique d'induction généré par la bobine de marquage (172a) induit par le champ magnétique variable.

24. Système médical de guidage magnétique selon la revendication 22, dans lequel l'unité (185) de stockage de données d'étalonnage stocke une pluralité de positions et de postures de l'unité (74) de variation de position/posture et les données d'étalonnage en fonction de la position et de la posture en association l'une avec l'autre entre celles-ci, l'unité (185) de stockage de données d'étalonnage obtient les données d'étalonnage en fonction des positions et des postures de l'unité (74) de variation de position/posture sur la base des données d'étalonnage stockées dans l'unité (185) de stockage de données d'étalonnage, et au moins une de la position et de la posture de l'unité (81) d'insertion est calculée sur la base de la sortie du capteur magnétique (126) et des données d'étalonnage obtenues par l'unité de calcul de position/posture.

25. Système médical de guidage magnétique selon la revendication 22, dans lequel la bobine d'excitation (181) est fixe relativement à l'unité (2) de génération de champ magnétique.

26. Système médical de guidage magnétique selon la revendication 22, dans lequel un cadre qui change la position de celui-ci par rapport à l'unité (2) de génération de champ magnétique par l'opération de l'unité (74) de variation de position/posture est prévu, et la bobine d'excitation (181) est fixée au cadre.

27. Système médical de guidage magnétique selon la revendication 1, dans lequel un noyau d'au moins un quelconque des trois électroaimants (3a, 3b, 4a, 4b, 5) comprend un élément ferromagnétique, et la superficie en coupe transversale sur le plan vertical au sens de magnétisation du noyau est maximum sur la surface d'extrémité proche du corps vivant (23).
